# EUROPÄISCHE PATENTANMELDUNG

(11) **EP 4 609 814 A1**
(43) Veröffentlichungstag der Anmeldung: **03.09.2025**
(21) Anmeldenummer: 25159806.6
(22) Anmeldetag: 25.02.2025
(51) Int. Cl.: A61B 34/20, A61B 34/30, A61B 90/20, A61B 90/00, A61B 34/00, A61F 9/007

(54) **STEUERVORRICHTUNG, ROBOTISCHE ANORDNUNG UND OPERATIONSMIKROSKOP FÜR DIE MIKROCHIRURGIE**

(30) Priorität: 28.02.2024 DE 102024105565
(71) Anmelder: Carl Zeiss Meditec AG, 07745 Jena (DE)
(72) Erfinder: BRENNER, Philipp, 73447 Oberkochen (DE); AMANOV, Ernar, 76344 Eggenstein-Leopoldshafen (DE); HILLENBRAND, Matthias, 73447 Oberkochen (DE); KLUSMANN, Carolin, 73447 Oberkochen (DE); REICHARD, Daniel, 76344 Eggenstein-Leopoldshafen (DE)
(74) Vertreter: PATERIS Patentanwälte PartmbB

(57) **Zusammenfassung**

Es wird eine Steuervorrichtung (1) für die Mikrochirurgie zur Steuerung eines chirurgischen Instruments (2) beschrieben. Die Steuervorrichtung (1) umfasst eine Sensoreinrichtung (3), welche mindestens eine Einrichtung (4) zum Erfassen der lateralen Position des chirurgischen Instruments (2) relativ zu einer Oberfläche (12) eines Gewebes (11) und mindestens eine Einrichtung (5) zum Erfassen von Daten, welche eine axiale Beziehung zwischen dem chirurgischen Instrument (2) und der Gewebeoberfläche (12) kennzeichnen. Die axiale Beziehung ist durch mindestens einen kinematischen und/oder mindestens einen dynamischen Parameter charakterisiert. Die Steuervorrichtung (1) ist zur Steuerung der Einwirkung des chirurgischen Instruments (2) auf das Gewebe (11) und/oder zur Steuerung der Bewegung des chirurgischen Instruments (2) in Bezug auf das Gewebe (11) basierend auf von der Sensoreinrichtung (3) erfassten Daten ausgelegt. Es wird zudem eine robotischen Anordnung und ein Operationsmikroskop für die Mikrochirurgie offenbart.

## Beschreibung

Die vorliegende Erfindung betrifft eine Steuervorrichtung, eine robotische Anordnung und ein Operationsmikroskop für die Mikrochirurgie, insbesondere für die Ophthalmochirurgie und die Neurochirurgie.

Aktuell werden chirurgische Verfahren typischerweise manuell ausgeführt. In bestimmten Gebieten, wie beispielsweise der Laparoskopie, gewinnen telemanipulierbare Operationsroboter an Popularität. In Zukunft werden Systeme mit einer Automatisierung auf Aufgabeneben oder mit einem höheren Grad an Automatisierung eine steigende Bedeutung erlangen. Entsprechende robotische Systeme werden eine an die Anforderungen angepasste, weiterentwickelte Sensorik und an die jeweils auszuführende Maßnahme angepasste Funktionen erfordern.

In vielen Verfahren der Ophthalmochirurgie müssen chirurgische Instrumente mit empfindlichem Gewebe in Kontakt gebracht werden, beispielsweise um bei einer Kataraktoperation einen Kapselsack zu greifen oder bei einem Membranpeeling in der Netzhautchirurgie eine Membran auf der Retina abzutragen. Die dabei optimalerweise wirkenden Kräfte liegen im Bereich weniger Millinewton (mN). Diese optimalen Kräfte zwischen der Spitze des chirurgischen Instruments und dem zu behandelnden Gewebe liegen oft unterhalb des für einen Menschen wahrnehmbaren Bereichs. Wenn die Kräfte zu hoch sind, besteht die Gefahr, dass das chirurgische Instrument umliegendes Gewebe beschädigt, z.B. die Retina während einer Netzhautoperation oder die Zonulafasern während einer Kataraktoperation. Sind die Kräfte zu gering, kann eine Durchführung des operativen Schritts nicht möglich sein, z.B. weil kein verlässliches Greifen eines Gewebes möglich ist. Ein Erfassen oder Fühlen der Kraft ist zudem deshalb kompliziert, weil während vieler augenchirurgischer Schritte Kräfte und Drehmomente am Eintrittspunkt in das Auge absorbiert werden, z.B. durch einen Trokar für die Netzhautchirurgie oder einen Schnitt in der Hornhaut im Rahmen einer Kataraktoperation. Im Ergebnis kann sich der/die Chirurg/in (im Folgenden auch Operateur genannt) während vieler ophthalmologischer Operationsschritte nicht auf ein haptisches Feedback verlassen und ist auf Sensoren außerhalb der eigenen Körperwahrnehmung angewiesen.

Ophthalmologische Operationsmikroskope eignen sich zum Erfassen der relativen lateralen Position zwischen einem chirurgischen Instrument und dem Gewebe und für eine grobe Abschätzung der Tiefe, z.B. der Eindringtiefe des Instruments in das Gewebe. Um eine unzureichende Kraft- und Tiefenerfassung zu kompensieren, verlassen sich Operateure häufig auf zusätzliche Hinweise, wie beobachtete Schatten, Farbveränderungen oder eine Faltenbildung des Gewebes unter Belastung. In anderen Fällen müssen Operationsschritte bei verschiedenen Tiefen wiederholt werden bis z.B. das Instrument greift und der chirurgische Schritt erfolgreich ausgeführt werden kann. Dies ist oft nur an Gewebereaktionen, wie z.B. der Ausbreitung eines Reißpunktes, erkennbar. Viele visuelle Hinweise, die ein Operateur typischerweise erfasst, sind mit einem Kamerasystem oder Computeralgorithmen schwer detektierbar.

Zur Unterstützung von Kataraktoperationen sind zum Beispiel Femtosekunden-Laser erhältlich. Diese Systeme weisen typischerweise einen integrierten optische Kohärenztomographen (OCT) für eine verbesserte Tiefensensorik auf. Hierdurch werden allerdings zusätzliche Kosten verursacht, auf dem Auge zu platzierende Kontaktgläser sind erforderlich und zudem kann der Kontakt zwischen dem chirurgischen Instrument und dem Gewebe unter Umständen nur unzureichend erfassbar sein.

In dem Dokument US 2011 / 0 106 102 A1 wird ein robotisches chirurgisches System für Augenoperationen beschrieben, wobei ein optischer Sensor im Bereich der Spitze eines chirurgischen Instruments genutzt wird. In dem Dokument US 2017 / 0 312 431 A1 wird ein augenchirurgisches System beschrieben, welches eine Druckmessung während einer Infusion ermöglicht. In dem Dokument US 2016 / 0 074 212 A1 wird eine Vorrichtung zum Einbringen eines Medikaments in ein Auge offenbart, welches ein Detektions- und Visualisierungssystem zur Detektion und Visualisierung einer Durchdringung der Aderhaut durch eine Injektionsnadel als Feedback für einen Operateur und/oder für eine automatisierte Steuerung umfasst.

In dem Dokument US 2007 / 0 151 390 A1 wird ein robotisches chirurgisches Instrument mit einem Kraft- und Drehmomentsensor an der Spitze beschrieben. In den Dokumenten US 2012 / 0 265 102 A1 und WO 2017 / 118949 A1 werden Herzkatheter mit Kraftsensoren an der Spitze offenbart.

Vor dem beschriebenen Hintergrund ist es Aufgabe der vorliegenden Erfindung eine vorteilhafte Steuervorrichtung für die Mikrochirurgie zur Steuerung eines chirurgischen Instruments zur Verfügung zu stellen. Weitere Aufgaben bestehen darin, eine vorteilhafte robotische Anordnung und ein Operationsmikroskop für die Mikrochirurgie zur Verfügung zu stellen.

Die genannten Aufgaben werden durch eine Steuervorrichtung gemäß Patenanspruch 1, eine robotische Anordnung gemäß Patentanspruch 7 und ein Operationsmikroskop gemäß Patentanspruch 15 gelöst. Die abhängigen Ansprüche enthalten weitere vorteilhafte Ausgestaltungen der Erfindung.

Die erfindungsgemäße Steuervorrichtung für die Mikrochirurgie zur Steuerung eines chirurgischen Instruments, insbesondere chirurgischen Werkzeugs oder Tools, umfasst eine Sensoreinrichtung und ist zur Steuerung des chirurgischen Instruments ausgelegt. Die Sensoreinrichtung umfasst mindestens eine Einrichtung zum Erfassen der lateralen Position des chirurgischen Instruments relativ zu einer Oberfläche eines Gewebes (Gewebeoberfläche) oder mit anderen Worten ein Mittel zum Erfassen der lateralen Position des chirurgischen Instruments in Bezug auf eine Gewebeoberfläche. Bei dem Gewebe kann es sich z.B. um Gewebe eines Auges eines Lebewesens, insbesondere eines Menschen, handeln. Die Sensoreinrichtung umfasst weiterhin mindestens eine Einrichtung zum Erfassen von Daten, welche eine axiale oder mediale Beziehung zwischen dem chirurgischen Instrument und der Gewebeoberfläche kennzeichnen. Dabei ist die axiale oder mediale Beziehung durch mindestens einen kinematischen und/oder mindestens einen dynamischen Parameter charakterisiert. Unter einer axialen oder medialen Beziehung zwischen dem chirurgischen Instrument und der Gewebeoberfläche wird eine Relation, z.B. ein räumlicher Abstand, oder eine Wechselwirkung zwischen dem chirurgischen Instrument und der Gewebeoberfläche in axialer oder medialer Richtung verstanden. Die axiale oder medialer Richtung verläuft vorzugsweise senkrecht zu einer durch zwei senkrecht zueinander angeordnete laterale Richtungen aufgespannten Ebene.

Bei dem mindestens einen kinematischen Parameter kann es sich z.B. um eine Ortskoordinate, eine Geschwindigkeit oder eine Beschleunigung des chirurgischen Instruments in Bezug auf die Gewebeoberfläche handeln. Bei dem mindestens einen dynamischen Parameter kann es sich z.B. um eine Krafteinwirkung des chirurgischen Instruments auf das Gewebe oder die Gewebeoberfläche handeln. Die Steuervorrichtung ist zur Steuerung einer Einwirkung, insbesondere einer Krafteinwirkung, des chirurgischen Instruments auf das Gewebe und/oder zur Steuerung der Bewegung des chirurgischen Instruments in Bezug auf das Gewebe, insbesondere in Bezug auf die Gewebeoberfläche, basierend auf von der Sensoreinrichtung erfassten Daten ausgelegt.

Unter einem chirurgischen Instrument wird vorliegend ein physisches Tool oder Werkzeug oder Gerät, mit welchem im Rahmen einer chirurgischen Maßnahme physisch auf Gewebe eingewirkt werden kann, verstanden.

Bei der Einrichtung zum Erfassen der lateralen Position des chirurgischen Instruments relativ zu einer Gewebeoberfläche und der Einrichtung zum Erfassen von Daten, welche eine axiale oder mediale Beziehung zwischen dem chirurgischen Instrument und der Gewebeoberfläche kennzeichnen, kann es sich um dieselbe Einrichtung oder verschiedene Einrichtungen handeln.

Die erfindungsgemäße Steuervorrichtung für die Mikrochirurgie ist vorzugsweise zur Steuerung eines chirurgischen Instruments während eines chirurgischen Verfahrens oder Eingriffs ausgelegt. Es kann sich um eine Steuervorrichtung für die Ophthalmochirurgie oder die Neurochirurgie handeln. Insbesondere kann die erfindungsgemäße Steuervorrichtung für einzelne Schritte, z.B. Kapsulorhexis oder Kapselsackpolieren, im Rahmen von Kataraktoperationen ausgelegt sein. Weitere Anwendungsbeispiele sind das Membran-Peeling in der Netzhautchirurgie, z.B. der Schritt des Bürstens zum Ablösen der abgerissenen Membran, die Hornhautchirurgie oder allgemein eine Vermeidung von zu hoher Krafteinwirkung im Rahmen von Phakoemulsifikation oder Linseninjektionen. Zudem kann die erfindungsgemäße Steuervorrichtung zum assistierten oder automatisierten Nähen in der Mikrochirurgie, z.B. in der Ophthalmochirurgie oder Neurochirurgie, ausgelegt sein.

Die erfindungsgemäße Steuervorrichtung hat den Vorteil, dass sie für mikrochirurgische Anwendungen ein verbessertes Feedback im Rahmen der Steuerung zu einer optimalen Durchführung des geplanten Eingriffs oder Verfahrens bietet. Die Steuerung ist damit sehr zuverlässig. Sie ist gleichzeitig kostengünstig, da keine kostenintensive Sensorik erforderlich ist.

Vorzugsweise handelt es sich bei dem mindestens einen kinematischen Parameter um den Abstand zwischen dem chirurgischen Instrument und der Gewebeoberfläche in einer axialen oder medialen Richtung. Es kann sich bei dem mindestens einen dynamischen Parameter um eine zwischen dem chirurgischen Instrument und dem Gewebe oder zwischen dem chirurgischen Instrument und der Gewebeoberfläche wirkende Kraft handeln.

Eine axiale oder mediale Beziehung zwischen dem chirurgischen Instrument und der Gewebeoberfläche kann auch als "Kontakt" verstanden werden, welcher definiert ist durch einen festgelegten Bereich einer zwischen dem chirurgischen Instrument und der Gewebeoberfläche wirkenden Kraft oder einen festgelegten Bereich eines räumlichen Abstandes zwischen dem chirurgischen Instrument und der Gewebeoberfläche. Dabei kann der festgelegte Bereich des räumlichen Abstandes zwischen dem chirurgischen Instrument und der Gewebeoberfläche positive Werte, negative Werte oder positive und negative Werte umfassen. Positive Werte treten auf, wenn sich das chirurgische Instrument über dem Gewebe, also von diesem beabstandet, befindet. Dies ist im Zusammenhang mit selbstschneidenden Sonden oder Werkzeugen, welche keine Kraft auf das Gewebe ausüben, relevant. Negative Werte treten auf, wenn das chirurgische Instrument das Gewebe eindrückt oder das chirurgische Instrument in das Gewebe eindringt. Bei einem Eindrücken kann sich z.B. die Kontur des umliegenden Gewebes verändern. Diese Veränderung kann mittels der Sensoreinrichtung erfasst werden und zur Steuerung des chirurgischen Instruments genutzt werden.

Die mindestens eine Einrichtung zum Erfassen der lateralen Position des chirurgischen Instruments relativ zu der Gewebeoberfläche kann mindestens eine Kamera, z.B. ein Operationsmikroskop, zum gleichzeitigen Erfassen einer Vielzahl an lateral angeordneten Bildpunkten und/oder eine Scanvorrichtung zum zeitsequenziellen Erfassen lateral angeordneter Bildpunkte umfassen. Die mindestens eine Einrichtung zum Erfassen der lateralen Position des chirurgischen Instruments relativ zu der Gewebeoberfläche und/oder die mindestens eine Einrichtung zum Erfassen einer medialen oder axialen Beziehung zwischen dem chirurgischen Instrument und der Gewebeoberfläche kann eine Stereokamera und/oder einen optischen Kohärenztomographen (OCT) umfassen. Hierbei können axiale Informationen mit niedriger Auflösung mit präziseren Informationen in Bezug auf die axiale Beziehung, zum Beispiel mit dem Abstand oder einer Krafteinwirkung zwischen dem chirurgischen Instrument und dem Gewebe, fusioniert oder verbunden oder ergänzt werden. Auf diese Weise lassen sich auf einfache und kostengünstige Art verlässliche axiale Daten zur Steuerung des chirurgischen Instruments gewinnen.

Weiterhin kann die mindestens eine Einrichtung zum Erfassen einer medialen oder axialen Beziehung zwischen dem chirurgischen Instrument und der Gewebeoberfläche mindestens einen in das chirurgische Instrument integrierten, z.B. eingebetteten, Sensor umfassen. Bei dem Sensor kann es sich um einen optischen frequenz-basierten Sensor handeln, zum Beispiel um einen Faser-Bragg-Gitter-Sensor mit in das chirurgische Instrument integrierten Fasern, einen Monomode-Faser-Hohlraum-Sensor oder ein Interferometer oder PIC (photonic integrated circuits), handeln. Es kann sich bei dem Sensor auch um einen in das chirurgische Instrument integrierten Dehnungsmessstreifen oder kapazitiven Sensor handeln. Weiterhin kann eine Faser mit einer in das chirurgische Instrument integrierten Optik zur Abstandsbestimmung vorhanden sein. Dies kann beispielsweise in Form eines OCT oder eines LIDAR-Sensors ausgestaltet sein. Es können auch Impedanz-Sensoren in das chirurgische Instrument integriert sein. Diese eignen sich insbesondere für eine verbesserte Steuerung der Eindringtiefe des chirurgischen Instruments in das Gewebe.

Zusätzlich oder alternativ dazu kann die Einrichtung zum Erfassen einer medialen oder axialen Beziehung zwischen dem chirurgischen Instrument und der Gewebeoberfläche mindestens einen Sensor in Kombination mit mindestens einem zusätzlichen Aktuator an einer Schnittstelle zwischen einem Roboterarm und dem chirurgischen Instrument umfassen. In diesem Zusammenhang kann eine oszillatorische Anregung des chirurgischen Instruments und eine Auswertung der Phasen- und/oder Frequenz-Antwort erfolgen. Es können auch Multiachsen-Kraft- und/oder Drehmomentsensoren in Verbindung mit einer Sensorik der RCM-Position (RCM - remote center of motion, z.B. Pivotpunkt im Einschnitt im Gewebe) und in Verbindung mit einem Kraft/Drehmoment-Modell vorhanden sein.

In einer weiteren zusätzlichen oder alternativen Variante kann die Einrichtung zum Erfassen einer medialen oder axialen Beziehung zwischen dem chirurgischen Instrument und der Gewebeoberfläche mindestens eine Einrichtung zum Erfassen von Gewebereaktionen und/oder mindestens eine Einrichtung zur Umwandlung oder Transformation von durch mechanische Spannung erzeugter mechanischer Verformung, z.B. elastischer Deformation, des chirurgischen Instruments, insbesondere von Teilbereichen des chirurgischen Instruments, in erfassbare optische Effekte umfassen. Erfassbare optische Effekte sind vorzugsweise mittels der Einrichtung zum Erfassen der lateralen Position des chirurgischen Instruments relativ zu dem Gewebe erfassbar.

Es können zum Beispiel die Reflexionen des Gewebes unter einer festgelegten Beleuchtung, welche festgelegte Muster aufweisen kann und/oder im nicht sichtbaren Wellenlängenbereich liegen kann, ausgewertet werden. Es kann auch die Polarisierung des Augengewebes unter Krafteinwirkung bzw. Belastung gemessen werden. Dies kann zum Beispiel räumlich aufgelöst oder polarimetrisch erfolgen. In einer weiteren Variante können Helligkeitsveränderungen in dem Gewebe in der Nähe eines Kontaktpunktes mit der Spitze des Instruments, also wenn die Spitze des chirurgischen Instruments auf das Gewebe gepresst wird, erfasst und ausgewertet werden. Eine geringe oder abgeschwächte Steifheit des chirurgischen Instruments, insbesondere eines bestimmten Bereichs des chirurgischen Instruments, kann zu einer makroskopisch beobachtbaren Deformation des chirurgischen Instruments führen, welche erfasst und ausgewertet werden kann. Auch kann sich die Polarisierung des chirurgischen Instruments in festgelegten Bereichen desselben bei Krafteinwirkung verändern. Auch dies kann polarimetrisch erfasst und ausgewertet werden. Es können insbesondere Moiré-Muster bei einer Veränderung der Position von mindestens zwei Bauelementen mit einem identischen Schachbrettmuster infolge einer mechanischen Deformation des chirurgischen Instruments erzeugt, erfasst und zur Bestimmung der axialen und/oder lateralen Beziehung zwischen dem chirurgischen Instrument und dem Gewebe ausgewertet werden. Weiterhin kann zusätzlich oder alternativ zu Moiré-Mustern eine Vernier-Skala verwendet werden. Alle bereits genannten Varianten können beliebig miteinander kombiniert werden.

Vorteilhafterweise ist die Steuervorrichtung zur Herbeiführung und/oder Aufrechterhaltung einer Kraftausübung, z.B. einer festlegbaren oder definierten Kraftausübung oder Krafteinwirkung, von dem chirurgischen Instrument auf das Gewebe ausgelegt. **In** einer weiteren Varianten kann die Steuervorrichtung zur Herbeiführung einer Kraftausübung von dem chirurgischen Instrument auf das Gewebe ausgelegt sein, wobei die ausgeübte Kraft einen festlegbaren oder festgelegten Schwellenwert nicht überschreitet. Hierdurch wird eine zu hohe Krafteinwirkung des chirurgischen Instruments auf das Gewebe vermieden.

Die Steuerung des chirurgischen Instruments kann in Abhängigkeit vom Vorliegen eines Abstandes zwischen dem chirurgischen Instrument und dem Gewebe oder der Gewebeoberfläche, z.B. eines Abstandes in medialer oder axialer Richtung, innerhalb eines von mehreren, z.B. mindestens zwei, festgelegten, voneinander verschiedenen Abstandsbereichen erfolgen. Es erfolgt also mit anderen Worten nur dann eine automatisierte oder assistierte Steuerung, wenn der Abstand zwischen dem chirurgischen Instrument und dem Gewebe oder der Gewebeoberfläche innerhalb eines festgelegten Bereichs liegt. Dabei kann der Abstand auch negativ sein, was einem Eindringen des chirurgischen Instruments in das Gewebe oder die Gewebeoberfläche entspricht.

Die erfindungsgemäße robotische Anordnung für die Mikrochirurgie, wobei es sich bei der Anordnung auch um ein robotisches System handeln kann, umfasst mindestens ein robotisch führbares, also robotisch bedienbares oder betätigbares, chirurgisches Instrument und mindestens einen robotischen Manipulator mit einer Befestigungseinrichtung zur Befestigung des chirurgischen Instruments an dem Manipulator. Die robotische Anordnung umfasst eine zuvor beschriebene erfindungsgemäße Steuervorrichtung, welche zur Steuerung des chirurgischen Instruments mittels des robotischen Manipulators ausgelegt ist. Die erfindungsgemäße robotische Anordnung hat dieselben Merkmale und Vorteile wie die zuvor beschriebene erfindungsgemäße Steuervorrichtung.

Der robotische Manipulator weist vorzugsweise mindestens 3 Bewegungsfreiheitsgrade zur Manipulation auf und die Steuervorrichtung ist vorzugsweise zur Steuerung der Bewegung des chirurgischen Instruments mittels der mindestens 3 Bewegungsfreiheitsgrade des robotischen Manipulators ausgelegt.

Vorteilhafterweise ist die robotische Anordnung so ausgelegt, dass als ein Bezugspunkt, z.B. im Form eines Koordinatenursprungs, für eine Bewegung des chirurgischen Instruments ein Eintrittspunkt in ein bestimmtes Gewebe, z.B. ein definiertes Gewebe eines bestimmten Organs, festgelegt oder festlegbar ist, wobei die laterale Position des chirurgischen Instruments mit einer Winkelausrichtung des chirurgischen Instruments oder des Manipulators in Bezug auf den Bezugspunkt, also mindestens einem Rotationswinkel des chirurgischen Instruments oder des Manipulators in Bezug auf den Bezugspunkt, und die axiale Position des chirurgischen Instruments mit einer Eintrittstiefe des chirurgischen Instruments oder des Manipulators durch den Eintrittspunkt korrespondiert.

Die robotische Anordnung kann zu einem teilautomatisierten oder vollautomatisierten Betrieb ausgelegt sein, wobei die lateralen Anteile einer Bewegungstrajektorie des chirurgischen Instruments in Bezug auf die Gewebeoberfläche basierend auf einer mittels der Sensoreinrichtung erfassten lateralen Position steuerbar sind und/oder der axiale oder mediale Anteil der Bewegungstrajektorie des chirurgischen Instruments in Bezug auf die Gewebeoberfläche basierend auf einer mittels der Sensoreinrichtung erfassten axialen oder medialen Beziehung zwischen dem chirurgischen Instrument und dem Gewebe steuerbar ist. Der Begriff Steuern umfasst dabei auch Regeln im Sinne der Steuerungs- und Regelungstechnik. Insbesondere kann ein Kontakt zwischen dem Gewebe und dem chirurgischen Instrument teilautomatisiert oder vollautomatisiert steuerbar sein. Hierbei kann die robotische Anordnung zu einer Regelung mittels eines Closed-Loop-Reglers zur Aufrechterhaltung eines Kontakts zwischen dem Gewebe und dem chirurgischen Instrument für eine festlegbare Zeitdauer ausgelegt sein.

In einer weiteren Variante kann die robotische Anordnung eine Mensch-Maschine-Schnittstelle umfassen, mittels welcher das chirurgische Instrument bezüglich seiner lateralen Bewegung, also in Bezug auf die lateralen Bewegungsanteile, in Bezug auf das Gewebe oder die Gewebeoberfläche durch einen Nutzer steuerbar ist, während eine axiale oder mediale Bewegung, also der axiale oder mediale Bewegungsanteil, des chirurgischen Instruments in Bezug auf das Gewebe oder die Gewebeoberfläche automatisiert steuerbar ist. Dabei kann die Steuerung voll- oder teilautomatisiert und/oder in Form einer Regelung erfolgen. Es kann beispielsweise eine Eingabevorrichtung, z.B. ein Joystick oder ähnliches, vorgesehen sein, mittels welcher ein Operateur oder Nutzer das chirurgische Instrument lateral steuern kann, während der Kontakt oder die axiale Beziehung zwischen dem chirurgischen Instrument und dem Gewebe basierend auf mittels der Sensoreinrichtung erfassten Daten aufrechterhalten wird. Die beschriebene Variante hat den Vorteil, dass eine zu große oder zu geringe Krafteinwirkung des chirurgischen Instruments auf das Gewebe vermieden wird, während gleichzeitig eine manuelle laterale Steuerung des chirurgischen Instruments ermöglicht wird.

Weiterhin kann die robotische Anordnung eine Mensch-Maschine-Schnittstelle mit einer Planungsfunktion, also eine entsprechende Komponente, umfassen, mittels welcher basierend auf mittels der Sensoreinrichtung erfassten lateralen Positionsdaten ein zu behandelnder Gewebebereich festlegbar ist, z.B. durch einen Nutzer, und die robotische Anordnung dazu ausgelegt ist, einen festgelegten chirurgischen Schritt teilautomatisiert oder automatisiert basierend auf mittels der Sensoreinrichtung erfassten Daten bezüglich der axialen oder medialen Beziehung zwischen dem chirurgischen Instrument und der Gewebeoberfläche auszuführen. Zum Beispiel kann mittels der Planungsfunktionen ein Operateur bzw. Nutzer einen zu behandelnden räumlichen Bereich basierend auf mittels der Sensoreinrichtung erfassten lateralen Positionsdaten definieren und die robotische Anordnung kann dazu ausgelegt sein, einen chirurgischen Schritt, beispielsweise das Greifen einer Kapselsack-Membran, an der benutzerdefinierten lateralen Position auszuführen, während das chirurgische Instrument für den Vorgang basierend auf mittels der Sensoreinrichtung erfassten Daten zu der axialen oder medialen Beziehung zwischen dem chirurgischen Instrument und dem Gewebe gebracht wird. Dabei kann eine Closed-Loop-Regelfunktionen vorgesehen oder vorhanden sein, welche dazu ausgelegt ist, das chirurgische Instrument automatisch in die richtige laterale Position und den richtigen Abstand zu dem Gewebe zu bringen.

Vorteilhafterweise umfasst die robotische Anordnung eine Mensch-Maschine-Schnittstelle, welche zur Ausgabe von Informationen zur Assistenz ausgelegt ist, insbesondere zur Ausgabe von Warnungen, Empfehlungen, z.B. zur Trajektorie des chirurgischen Instruments. Auch in diesem Zusammenhang kann eine Closed-Loop-Regelung vorgesehen sein.

In einer weiteren Variante kann die robotische Anordnung während der Ausführung einer chirurgischen Maßnahme oder Aufgabe zwischen zwei Betriebsmodi umschaltbar sein, wobei in einem ersten Betriebsmodus das chirurgische Instrument und das Gewebe im Kontakt sind und in einem zweiten Betriebsmodus das chirurgische Instrument und das Gewebe nicht im Kontakt sind. Der zweite Betriebsmodus kann zur Repositionierung des chirurgischen Instruments ausgelegt sein oder hierzu genutzt werden. Die Auswahl des Betriebsmodus kann basierend auf von der Sensoreinrichtung erfassten Daten erfolgen. Die Betriebsmodi können manuell und/oder automatisiert umschaltbar sein. Im Rahmen der Betriebsmodi können Closed-Loop-Regelungen vorgesehen sein. Zum Beispiel kann in dem ersten Betriebsmodus eine Membran zum Abreißen dieser durch das chirurgische Instrument gegriffen werden und in dem zweiten Betriebsmodus kann die Membran losgelassen werden, um das chirurgische Instrument zu repositionieren. In beiden Betriebsmodi liefert die Sensoreinrichtung oder die Sensoreinrichtungen die erforderlichen Informationen, um die angestrebte axiale oder mediale Beziehung zwischen dem chirurgischen Instrument und dem Gewebe aufrechtzuerhalten. Das Umschalten kann beispielsweise basierend auf lateralen Positions-Informationen manuell erfolgen oder basierend auf dem Fortschritt eines chirurgischen Schrittes und Informationen von der zur lateralen Positionsauslesung vorgesehenen Sensoreinrichtung automatisiert erfolgen.

Die robotische Anordnung kann dazu ausgebildet sein, eine Bewegung des Gewebes zu erfassen und im Falle einer erfassten Bewegung des Gewebes, zum Beispiel infolge von Herzschlag oder Atmung, mindestens das chirurgische Instrument, gegebenenfalls auch weitere Komponenten, der erfassten Bewegung nachzuführen. Das Nachführen kann automatisiert, z.B. teilautomatisiert oder vollautomatisiert, ausgelegt sein bzw. erfolgen. Das Nachführen kann durch festlegbare oder festgelegte Grenzen beschränkt sein. Das Erfassen einer Bewegung des Gewebes kann mittels der Sensoreinrichtung oder weiterer Sensoreinrichtungen, z.B. einem Head-Tracker oder einem Eye-Tracker, erfolgen. Die robotische Anordnung kann zum Erfassen einer Bewegung des Gewebes in lateraler und/oder axialer Richtung ausgelegt sein. Zudem kann die robotische Anordnung zum Nachführen des chirurgischen Instruments und gegebenenfalls weiterer Komponenten in lateraler und/oder axialer Richtung ausgelegt sein.

Das erfindungsgemäße Operationsmikroskop für die Mikrochirurgie umfasst eine oben beschriebene erfindungsgemäße Steuervorrichtung oder eine zuvor beschriebene erfindungsgemäße robotische Anordnung. Das erfindungsgemäße Operationsmikroskop hat die bereits im Zusammenhang mit der erfindungsgemäßen Steuervorrichtung und der erfindungsgemäßen robotische Anordnung beschriebenen Merkmale und Vorteile.

Im Folgenden werden beispielhafte Ausführungsvarianten für Assistenzfunktionen unter Nutzung der erfindungsgemäßen Steuervorrichtung bei einer nicht-robotischen Anwendung, also einer manuellen Steuerung des chirurgischen Instruments unter Verwendung der erfindungsgemäßen Steuervorrichtung beschrieben. Wenn die Sensoreinrichtung Daten zu einer Krafteinwirkung zwischen dem Gewebe und dem chirurgischen Instrument liefert, kann das chirurgische Setting durch ein Kraft-Feedback erweitert werden. Sobald mittels einer der beschriebenen Verfahren eine Kraft gemessen wird, kann ein Bereich um die Spitze des chirurgischen Instruments eingefärbt angezeigt werden. Die Farbe kann sich kontinuierlich zwischen verschiedenen Farben in Abhängigkeit von der Krafteinwirkung ändern. Zum Beispiel kann gelb für eine sehr geringe Krafteinwirkung, grün für eine gute oder optimale Krafteinwirkung und rot für eine zu hohe Krafteinwirkung verwendet werden. Im Falle der Verwendung von Abstandsmessungsverfahren, wie beispielsweise einem integrierten OCT A-Scan Ansatz, kann die Einfärbung bereits erfolgen bevor eine Wechselwirkung zwischen dem chirurgischen Instrument und dem Gewebe stattfindet. Es kann ein akustisches Feedback mittels verschiedener Tonhöhen oder Frequenzen von sich wiederholenden Tönen zur Verfügung gestellt werden. Falls die Krafteinwirkung aus einer Gewebe-Deformation abgeleitet wird, kann das Gewebe als Landkarte oder Heatmap eingefärbt werden, wobei die Tiefe jedes Punktes des deformierten Bereichs farblich gekennzeichnet ist.

Falls im Falle einer manuellen Steuerung der Abstand zwischen dem chirurgischen Instrument und im Gewebe erfasst wird, ist ein entsprechendes Tiefen-Feedback möglich. Sobald ein Kontakt zwischen dem chirurgischen Instrument und dem Gewebe bzw. der Gewebeoberfläche erfasst wird, kann der Bereich um die Spitze des chirurgischen Instruments herum gefärbt angezeigt werden. Es kann zusätzlich oder alternativ dazu ein akustisches Feedback, wie bereits beschrieben, zur Verfügung gestellt werden.

Im Folgenden werden beispielhafte Ausführungsvarianten für Assistenzfunktionen unter Nutzung der erfindungsgemäßen Steuervorrichtung und/oder der erfindungsgemäßen robotischen Anordnung für eine robotische teilautomatisierte Steuerung des chirurgischen Instruments beschrieben. Dabei ergeben sich zusätzlich zu den bereits genannten Optionen für eine manuelle Steuerung bei erfassten Daten bezüglich der Kraft zwischen dem chirurgischen Instrument und dem Gewebe folgende Optionen: Es kann dem Operateur über eine Mensch-Maschine-Schnittstelle ein haptisches Feedback in Form von Vibrationen oder aktivem Widerstand in der entsprechenden Bewegungsrichtung zur Verfügung gestellt werden. Dabei können die Intensität und das Ausmaß der Vibration und/oder des Widerstandes von dem Ausmaß der erfassten oder gemessenen oder abgeleiteten Kraft abhängen. In einer zweiten Option kann die robotische Bewegung in axialer Richtung (z-Richtung), also in medialer Richtung in Bezug auf das Gewebe, beschränkt sein, sodass eine weitere ein weiteres Eindringen in das Gewebe vermieden wird. Auch kann vorgesehen sein, dass keine weitere Nutzereingabe möglich ist, solange die Einwirkung des chirurgischen Instruments auf das Gewebe ausreichend ist. In einer dritten Option kann das chirurgische Instrument automatisiert in axialer Richtung, also nach oben und unten, bewegt werden, um einen ausreichenden Griff oder eine anderweitige ausreichende Einwirkung zu erzielen, sodass der Operateur lediglich eine Steuerung in lateraler Richtung, also eine Steuerung der lateralen Bewegung des chirurgischen Instruments, vornehmen kann.

Im Falle einer Erfassung des axialen oder medialen Abstandes zwischen dem chirurgischen Instrument und dem Gewebe sind folgende Optionen möglich: Es kann ein haptisches Feedback in Abhängigkeit von dem erfassten Abstand analog zu der erfassten Kraft, wie oben beschrieben, zur Verfügung gestellt werden. Es kann, ebenfalls analog zur Abhängigkeit von einer erfassten Krafteinwirkung, auch in Abhängigkeit von einem erfassten Abstand die Bewegung des chirurgischen Instruments in axialer oder medialer Richtung beschränkt sein, und es kann eine in Abhängigkeit von dem erfassten Abstand automatisierte Bewegung des chirurgischen Instruments in axialer bzw. medialer Richtung erfolgen, sodass der Operateur lediglich eine Steuerung der lateralen Bewegung des chirurgischen Instruments vornehmen kann.

Die Erfindung wird im Folgenden anhand von Ausführungsbeispielen unter Bezugnahme auf die beigefügten Figuren näher erläutert. Obwohl die Erfindung im Detail durch die bevorzugten Ausführungsbeispiele näher illustriert und beschrieben wird, so ist die Erfindung nicht durch die offenbarten Beispiele eingeschränkt und andere Variationen können vom Fachmann hieraus abgeleitet werden, ohne den Schutzumfang der Erfindung zu verlassen.

Die Figuren sind nicht notwendigerweise detailgetreu und maßstabsgetreu und können vergrößert oder verkleinert dargestellt sein, um einen besseren Überblick zu bieten. Daher sind hier offenbarte funktionale Einzelheiten nicht einschränkend zu verstehen, sondern lediglich als anschauliche Grundlage, die dem Fachmann auf diesem Gebiet der Technik Anleitung bietet, um die vorliegende Erfindung auf vielfältige Weise einzusetzen.

Der hier verwendete Ausdruck "und/oder", wenn er in einer Reihe von zwei oder mehreren Elementen benutzt wird, bedeutet, dass jedes der aufgeführten Elemente alleine verwendet werden kann, oder es kann jede Kombination von zwei oder mehr der aufgeführten Elemente verwendet werden. Wird beispielsweise eine Zusammensetzung beschrieben, die die Komponenten A, B und/oder C, enthält, kann die Zusammensetzung A alleine; B alleine; C alleine; A und B in Kombination; A und C in Kombination; B und C in Kombination; oder A, B, und C in Kombination enthalten.
- Fig. 1: zeigt schematisch eine erfindungsgemäße Steuervorrichtung in Form eines Blockdiagramms.
- Fig. 2: zeigt schematisch eine erfindungsgemäße robotische Anordnung in Form eines Blockdiagramms.
- Fig. 3: zeigt schematisch ein erfindungsgemäßes Operationsmikroskop in Form eines Blockdiagramms.
- Fig. 4: zeigt schematisch einen Teilbereich eines chirurgischen Instruments mit einem integrierten Faser-Bragg-Gitter-Sensor.
- Fig. 5: zeigt schematisch das Wirkungsprinzip eines in ein Instrument integrierten Interferometers.
- Fig. 6 und 7: zeigen schematisch Dehnungsstreifen.
- Fig. 8 und 9: zeigen schematisch die Wirkungsweise eines kapazitiven Sensors.
- Fig.10: zeigt schematisch die Spitze eines chirurgischen Instruments mit einem integrierten kapazitiven Sensor.
- Fig. 11: zeigt schematisch die Abhängigkeit der gemessenen Kapazität von einer Berührung des kapazitiven Sensors.
- Fig. 12: zeigt schematisch eine weitere Variante eines kapazitiven Sensors.
- Fig.13: zeigt schematisch die Spitze eines chirurgischen Instruments mit einem integrierten kapazitiven Sensor in einer Variante.
- Fig. 14: zeigt schematisch einen Teilbereich eines chirurgischen Instruments in einer längsgeschnittenen Ansicht.
- Fig. 15: zeigt schematisch einen Teilbereich eines chirurgischen Instruments in einer quergeschnittenen und perspektivischen Ansicht.
- Fig. 16: zeigt schematisch das Prinzip einer optischen Abstandsmessung.
- Fig. 17: zeigt schematisch ein in einer Nadelhülle angeordnetes Stilett.
- Fig. 18: zeigt schematisch das Prinzip eines in ein chirurgisches Instrument integrierten Impedanz-Sensors.
- Fig. 19: zeigt schematisch ein chirurgisches Instrument mit integriertem Impedanz-Sensor.
- Fig. 20: zeigt schematisch eine weitere Variante eines chirurgischen Instruments mit integriertem Impedanz-Sensor.
- Fig. 21: zeigt schematisch das Prinzip der Erfassung eines Kontakts basierend auf einer zusätzlichen Anregung des Sensors an einer Schnittstelle zwischen einem robotischen Arm und dem chirurgischen Instrument.
- Fig. 22: zeigt schematisch die Vibration der Spitze eines chirurgischen Instruments bei der Erfassung eines Kontakts.
- Fig. 23: zeigt schematisch in ein Verbindungselement zwischen einem robotischen Arm und dem chirurgischen Instrument integrierte kapazitive Sensoren.
- Fig. 24: zeigt schematisch die Transmission von Licht in Abhängigkeit von der Wellenlänge durch verschiedene Gewebe eines Auges.
- Fig. 25: zeigt schematisch ein mittels strukturierter Beleuchtung auf einem Gewebe erzeugtes Muster.
- Fig. 26: zeigt schematisch ein Augengewebe und ein darauf angeordnetes chirurgisches Instrument.
- Fig. 27: zeigt schematisch ein Augengewebe und ein darauf einwirkendes chirurgisches Instrument.
- Fig. 28: zeigt schematisch ein Beispiel für ein chirurgisches Instrument mit einem geschwächten Bereich oder einem Gelenk.
- Fig. 29: zeigt schematisch einen Teilbereich eines chirurgischen Instruments mit einem geschwächten Bereich.
- Fig. 30: zeigt schematisch ein Augengewebe und ein darauf einwirkendes chirurgisches Instrument in einer Draufsicht.
- Fig. 31: zeigt schematisch das in der Figur 30 gezeigte Augengewebe und das auf dieses einwirkende chirurgische Instrument in einer Seitenansicht.
- Fig. 32: zeigt schematisch ein Augengewebe und ein darauf einwirkendes chirurgisches Instrument in einer Seitenansicht.
- Fig. 33: zeigt schematisch einen zur Deformation ausgelegten Bereich eines chirurgischen Instruments in einer Draufsicht.
- Fig. 34: zeigt schematisch das Prinzip einer polarimetrischen Bestimmung der bei Krafteinwirkung in einem Werkzeug auftretenden Spannung.
- Fig. 35: zeigt schematisch Moiré-Muster.
- Fig. 36: zeigt schematisch einen Teilbereich eines Messschiebers zur Veranschaulichung der Vernier-Skala.

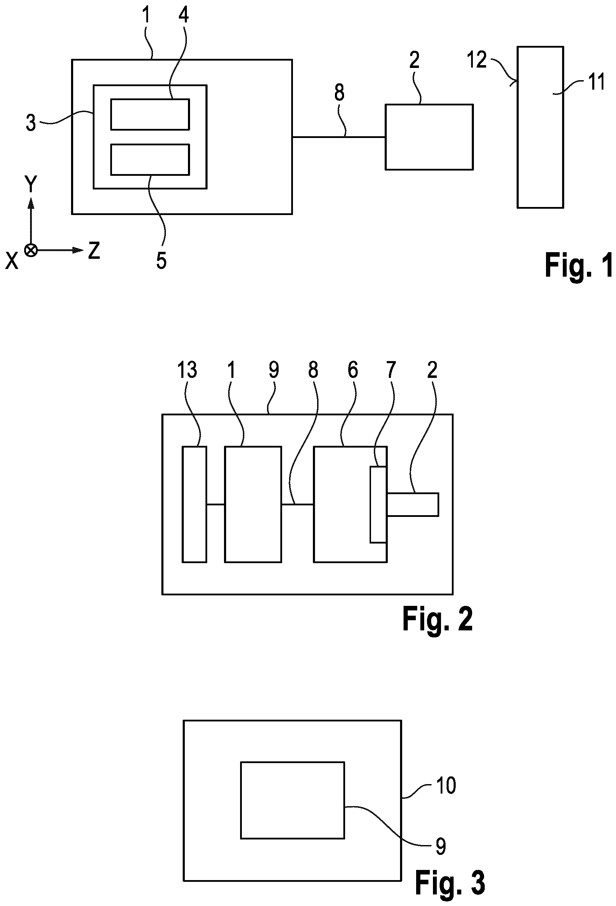

Die Figur 1 zeigt schematisch eine erfindungsgemäße Steuervorrichtung in Form eines Blockdiagramms. Die Steuervorrichtung 1 ist für die Mikrochirurgie zur Steuerung eines chirurgischen Instruments 2 ausgelegt. In der Figur 1 kennzeichnet die mit der Bezugsziffer 8 gekennzeichnete Linie die Steuerung des chirurgischen Instruments 2 mittels der Steuervorrichtung 1, also eine Einwirkung auf das chirurgische Instrument 2. Die Einwirkung auf das chirurgische Instrument 2 kann beispielsweise vollautomatisiert oder teilautomatisiert oder assistiert manuell erfolgen.

Die Steuervorrichtung 1 umfasst eine Sensoreinrichtung 3. Die Sensoreinrichtung 3 umfasst mindestens eine Einrichtung 4 zum Erfassen der lateralen Position des chirurgischen Instruments 2 relativ zu einer Oberfläche 12 eines Gewebes 11, zum Beispiel des Gewebes eines Auges. Die Sensoreinrichtung 3 umfasst zudem mindestens eine Einrichtung 5 zum Erfassen von Daten, welche eine axiale Beziehung zwischen dem chirurgischen Instrument 2 und der Gewebeoberfläche 12 kennzeichnen. Dabei ist die axiale Beziehung durch mindestens einen kinematischen und/oder mindestens einen dynamischen Parameter charakterisiert. Die Steuervorrichtung 1 ist zur Steuerung der Einwirkung des chirurgischen Instruments 2 auf das Gewebe 11 und/oder zur Steuerung der Bewegung des chirurgischen Instruments 2 in Bezug auf das Gewebe 11 basierend auf von der Sensoreinrichtung 3 erfassten Daten ausgelegt.

Bei dem kinematischen Parameter kann es sich um den Abstand zwischen dem chirurgischen Instrument 2 und der Gewebeoberfläche 12 in einer axialen oder medialen Richtung handeln. In der Figur 1 entspricht die axiale oder mediale Richtung einer Richtung entlang der z-Achse des gezeigten Koordinatensystems. Laterale Richtungen verlaufen entlang der x-Achse und/oder der y-Achse des gezeigten Koordinatensystems.

Die mindestens eine Einrichtung 4 zum Erfassen der lateralen Position chirurgischen Instruments 2 bezüglich der Gewebeoberfläche 12 kann mindestens eine Kamera, beispielsweise eine Kamera eines Operationsmikroskops, zum gleichzeitigen Erfassen einer Vielzahl an lateralen angeordneten Bildpunkten umfassen. Zusätzlich oder alternativ dazu kann die Einrichtung 4 eine Scanvorrichtung zum zeitsequenziellen Erfassen lateral angeordneter Bildpunkte umfassen.

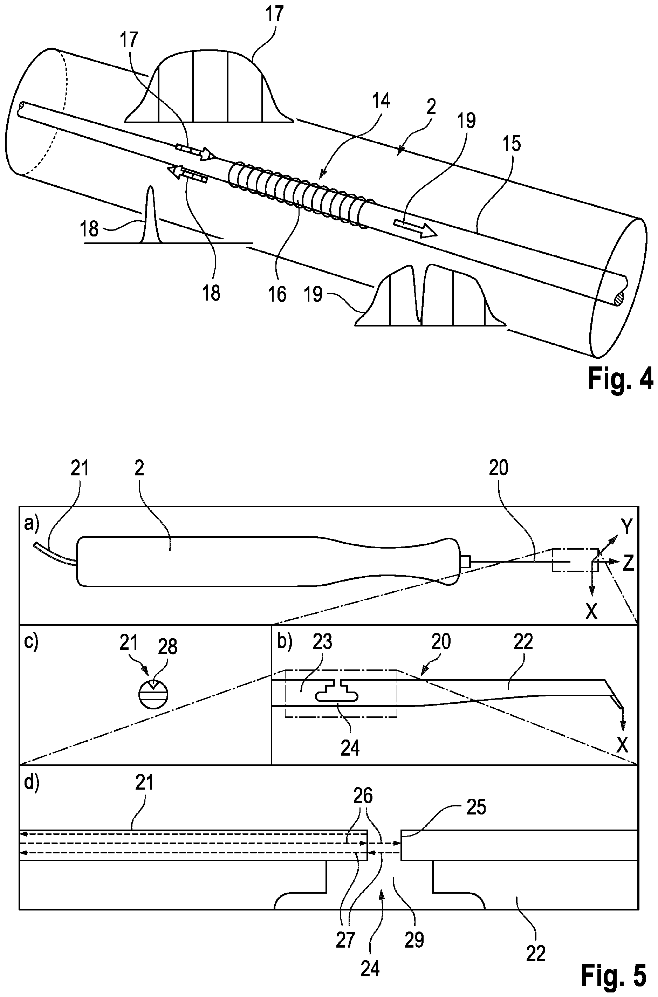

Die Einrichtung 4 und/oder die Einrichtung 5 können eine Stereokamera und/oder einen optischen Kohärenztomographen (OCT) umfassen. Die Einrichtung 4 und/oder die Einrichtung 5 können zumindest teilweise in das mindestens eine chirurgische Instrument 2 integriert sein. Das chirurgische Instrument 2 kann also mindestens einen Sensor aufweisen. Mögliche Ausgestaltungen geeigneter Sensoren werden unten anhand der Figuren 4ff. beschrieben.

Die Steuervorrichtung 1 kann zur Herbeiführung und/oder Aufrechterhaltung einer Kraftausübung von dem chirurgischen Instrument 2 auf das Gewebe 11 oder die Oberfläche 12 des Gewebes 11 ausgelegt sein. Die Steuervorrichtung kann zur Herbeiführung einer Kraftausübung von dem chirurgischen Instrument 2 auf das Gewebe 11 oder die Gewebeoberfläche 12 ausgelegt sein, wobei die ausgeübte Kraft einen festlegbaren Schwellenwert nicht überschreitet.

Die Figur 2 zeigt schematisch eine erfindungsgemäße robotische Anordnung 9 für die Mikrochirurgie in Form eines Blockdiagramms. Die robotische Anordnung 9 umfasst mindestens ein robotisch führbares chirurgisches Instrument 2 und mindestens einen robotischen Manipulator 6. Der robotische Manipulator 6 weist eine Befestigungseinrichtung 7 zur Befestigung des chirurgischen Instruments 2 an dem Manipulator 6 auf. Die robotische Anordnung 9 umfasst eine im Zusammenhang mit der Figur 1 beschriebene Steuervorrichtung 1. Die Steuervorrichtung 1 ist zur Steuerung des chirurgischen Instruments mittels des robotischen Manipulators 6 ausgelegt. Die Steuerung des robotischen Manipulators mittels der Steuervorrichtung 1 ist durch die Bezugsziffer 8 gekennzeichnet.

Der robotische Manipulator 6 kann mindestens drei Bewegungsfreiheitsgrade zur Manipulation aufweisen und die Steuervorrichtung 1 kann zur Steuerung der Bewegung des chirurgischen Instruments 2 mittels der mindestens drei Bewegungsfreiheitsgrade des robotischen Manipulators 6 ausgelegt sein. Die robotische Anordnung kann so ausgelegt sein, dass als ein Bezugspunkt für eine Bewegung des chirurgischen Instruments 2 ein Eintrittspunkt in ein bestimmtes Gewebe 11 festgelegt oder festlegbar ist, wobei die laterale Position des chirurgischen Instruments 2 mit einer Winkelausrichtung des chirurgischen Instruments 2 oder des Manipulators 6 und die axiale Position des chirurgischen Instruments 2 mit einer Eintrittstiefe des chirurgischen Instruments 2 oder des Manipulators 6 durch den Eintrittspunkt korrespondiert.

Die robotische Anordnung 9 kann zu einem teilautomatisierten oder vollautomatisierten Betrieb ausgelegt sein, wobei die lateralen Anteile einer Bewegungstrajektorie des chirurgischen Instruments 2 in Bezug auf die Gewebeoberfläche 12 basierend auf einer mittels der Sensoreinrichtung 1 erfassten lateralen Position steuerbar sind und/oder der axiale Anteil der Bewegungstrajektorie des chirurgischen Instruments 2 in Bezug auf die Gewebeoberfläche 12 basierend auf einer mittels der Sensoreinrichtung 1 erfassten axialen oder medialen Beziehung zwischen dem chirurgischen Instrument 2 und dem Gewebe 11 steuerbar ist. Die robotische Anordnung 9 kann eine Mensch-Maschine-Schnittstelle 13 umfassen, mittels welcher das chirurgische Instrument 2 bezüglich seiner lateralen Bewegung in Bezug auf das Gewebe 11 oder die Gewebeoberfläche 12 durch einen Operateur steuerbar ist, während eine mediale oder axiale Bewegung des chirurgischen Instruments 2 in Bezug auf das Gewebe 11 oder die Gewebeoberfläche 12 automatisiert steuerbar ist.

Die Mensch-Maschine-Schnittstelle 13 kann eine Planungsfunktion aufweisen, mittels welcher basierend auf mittels der Sensoreinrichtung 3 erfassten lateralen Positionsdaten ein zu behandelnder Gewebebereich festlegbar ist. Die robotische Anordnung 9 kann dazu ausgelegt sein, mindestens einen festgelegten chirurgischen Schritt teilautomatisiert oder automatisiert basierend auf mittels der Sensoreinrichtung 1 erfassten Daten bezüglich der axialen Beziehung zwischen dem chirurgischen Instrument 2 und der Gewebeoberfläche 12 auszuführen. Die Mensch-Maschine-Schnittstelle 13 kann zur Ausgabe von Informationen zur Assistenz ausgebildet sein. Die Ausgabe kann visuell und/oder akustisch und/oder haptisch erfolgen.

In einer weiteren Varianten ist die robotische Anordnung 9 während der Ausführung einer chirurgischen Maßnahme zwischen zwei Betriebsmodi umschaltbar, wobei in einem ersten Betriebsmodus das chirurgische Instrument 2 und das Gewebe 11 im unmittelbaren Kontakt sind und in einem zweiten Betriebsmodus das chirurgische Instrument 2 und das Gewebe 11 nicht im unmittelbaren Kontakt sind. Die robotische Anordnung 9 kann weiterhin dazu ausgebildet sein, eine Bewegung des Gewebes 11 zu erfassen und im Falle einer erfassten Bewegung des Gewebes 11 mindestens das chirurgische Instrument 2 der erfassten Bewegung nachzuführen. **In** weiteren Varianten kann die robotische Anordnung 9 zu einem automatisierten Instrumentenwechsel ausgelegt sein. Weiterhin kann die robotische Anordnung 9 zu einer Zuleitung, z.B. einer automatisierten Zuleitung von Licht und/oder Flüssigkeiten ausgebildet sein. Die Anschlüsse dafür können in die Befestigungseinrichtung 7 integriert sein.

Die Figur 3 zeigt schematisch ein erfindungsgemäßes Operationsmikroskop 10 in Form eines Blockdiagramms. Das Operationsmikroskop 10 ist für die Mikrochirurgie ausgelegt und umfasst eine im Zusammenhang mit der Figur 2 beschriebene robotische Anordnung 9.

Im Folgenden werden Ausgestaltungsoptionen für die Sensorik zum Erfassen von Daten, welche eine axiale Beziehung zwischen dem chirurgischen Instrument 2 und der Gewebeoberfläche 12 charakterisieren, und/oder zum Erfassen der lateralen Position des chirurgischen Instruments 2 relativ zu einer Gewebeoberfläche 12 beschrieben. Dabei werden zunächst in ein chirurgisches Instrument integrierte Sensoren zur Kontakterfassung beschrieben. Als optische frequenzbasierte bzw. interferometrische Sensoren eignen sich dabei Faser-Bragg-Gitter-Sensoren oder Interferometer.

Die Figur 4 zeigt schematisch einen Teilbereich eines chirurgischen Instruments 2 mit einem integrierten Faser-Bragg-Gitter-Sensor 14 in einer perspektivischen Ansicht sowie dessen Wirkungsweise. Dabei werden Deformationen des chirurgischen Instruments 2 unter Verwendung von optischen Fasern 15 mit integrierten optischen Gittern 16 gemessen dabei können die Gitter 16 die Krümmungen an diskreten Punkten abbilden. Eine Faser 15 kann viele dieser Gitter 16 in seine geometrische Form integriert aufweisen. Mindestens eine Faser 15 muss für eine eindimensionale Messung integriert vorhanden sein. Mit diesem Ansatz kann allerdings lediglich die Krümmung gemessen werden, nicht jedoch die Richtung einer Biegung des chirurgischen Instruments. Für eine zweidimensionale Messung sind 2-3 Fasern erforderlich und für eine Messung einer Verdrehung des chirurgischen Instruments, also einer Verdrillung um die Längsachse, ist eine weitere Faser erforderlich. Zur Reduzierung der Kosten ist die Verwendung einer Faser 15 vorzuziehen. Das Sensor-Ausgangssignal kann in diesem Fall mit einem Ausgangssignal einer Kamera von einem Mikroskop kombiniert werden, welches dazu ausgelegt ist laterale Deformationen zu erfassen oder zu messen.

Zur Bestimmung einer Krümmung eines chirurgischen Instruments 2 mittels des Faser-Bragg-Gitter-Sensors 14 wird ein breitbandiges Licht auf das oder die Gitter 16 in Längsrichtung der Faser 15 eingestrahlt. Die Einstrahlrichtung und das Frequenzspektrum des eingestrahlten Lichts sind in der Figur 4 schematisch mit der Bezugsziffer 17 gekennzeichnet. Ein Teil des Lichts wird von den Gittern 16 reflektiert. Die Strahlrichtung und das Frequenzspektrum des reflektierten Lichts sind in der Figur 4 schematisch mit der Bezugsziffer 18 gekennzeichnet. Das durch das Gitter 16 transmittierte Licht ist in Bezug auf seine Ausbreitungsrichtung und sein Frequenzspektrum in der Figur 4 mit der Bezugsziffer 19 gekennzeichnet. Durch Auswertung des reflektierten oder transmittierten Frequenzspektrums lässt sich die Krümmung des chirurgischen Instruments 2 ermitteln.

Die Figur 5 zeigt schematisch das Wirkungsprinzip eines in ein Instrument 2 integrierten Interferometers. Das in der Figur 5a) beispielhaft gezeigte Instrument 2 weist eine Spitze 20 auf, welche in der Figur 5b) vergrößert dargestellt ist. Mindestens eine beschichtete Faser 21 ist in das Instrument 2 und die Spitze 20 integriert. Die Figur 5c) zeigt die beschichtete optische Faser 21 in einem Querschnitt, wobei einzelne Fasern mit der Bezugsziffer 28 gekennzeichnet sind. Die Spitze 20 umfasst, wie in den Figuren 5b) und 5d) gezeigt, einen festen oder steifen Bereich 22 der Spitze und einen Basisbereich 23 der Spitze auf, welche in Längsrichtung miteinander durch einen biegbaren Verbindungsbereich 24 verbunden sind. Der Verbindungsbereich 24 umfasst einen Hohlraum 29 zwischen dem Basisbereich 23 und dem festen Bereich 22. Der biegbare Bereich 24 ist in der Figur 5d) vergrößert dargestellt. Auf den festen Bereich 22 wirkende Kräfte, welche zu einer mechanischen Deformation des Verbindungsbereichs 24 führen, bewirken eine Veränderung der Länge des Hohlraums 29 in Längsrichtung.

Die Faser 21 endet in dem in den Verbindungsbereich 24 hineinreichenden Basisbereich 23. Der biegbare Bereich 24 weist dabei an dem festen Bereich 22 eine reflektive Oberfläche 25 auf. Zur Bestimmung der Biegung oder Krümmung der Spitze 20 wird mittels der optischen Faser oder der Fasern 21 auf die reflektive Oberfläche 25 eingestrahltes und von dieser reflektiertes Licht ausgewertet. Dabei wird eine Veränderung der Länge des Hohlraums 29 in Längsrichtung optisch erfasst, beispielsweise durch eine Auswertung der Resonanzfrequenz des Hohlraums. Weitere Details zu dem beschriebenen Verfahren sind in dem Dokument Fifanski et al.: VivoForce instrument for retinal microsurgery, Surgetica 2017 offenbart. In der Figur 5d) ist das eingestrahlte Licht mit der Bezugsziffer 26 und das reflektierte Licht mit Bezugsziffer 27 gekennzeichnet.

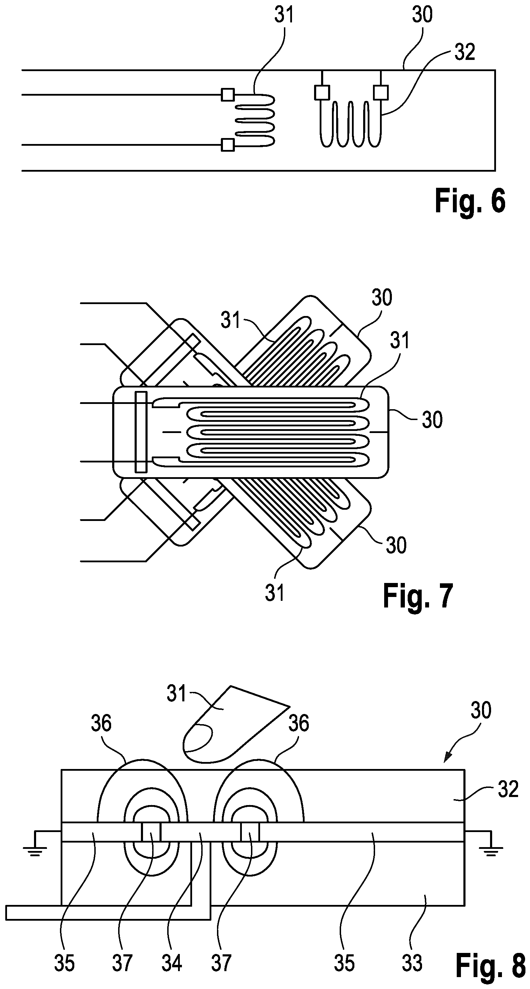

Die Figuren 6 und 7 zeigen schematisch Dehnungsstreifen 30, welche in ein chirurgisches Instrument 2 integriert werden können. Diese können, wie in der Figur 6 gezeigt, nebeneinander angeordnete Schaltkreise bzw. Stromkreise aufweisen oder, wie in der Figur 7 gezeigt, aufeinander angeordnet sein, um eine Dehnung bzw. Deformationen des chirurgischen Instruments 2 in verschiedenen Richtungen zu erfassen. Dehnungsstreifen sind zuverlässige Standardgeräte zur Kraftmessung. Bei einer Integration in ein chirurgisches Instrument sollte das chirurgische Instrument 2 einen Kern aus nichtleitendem Material aufweisen, welcher mit einer leitfähigen Schicht bedeckt ist. Dehnungsschaltkreise 31 und 32 können, beispielsweise durch LaserSchneiden, auf dem der leitfähigen Schicht, wie in den Figuren 6 und 7 gezeigt, erzeugt werden. Die Anzahl und Position der Schaltkreise 31, 32 in oder auf dem chirurgischen Instrument 2 ist entscheidend für die Qualität und die räumliche Auflösung der Messung entlang des chirurgischen Instruments 2. Das nichtleitfähige Material sollte so gewählt sein, dass es in Bezug auf die erwarteten Deformationen perfekt elastisch ausgebildet ist. Die Verbindung zwischen zwei Lagen oder Schichten muss so fest sein, sodass die tatsächliche Deformation des Instruments 2 und nicht die Deformationen eines die verschiedenen Lagen verbindenden Materials gemessen wird.

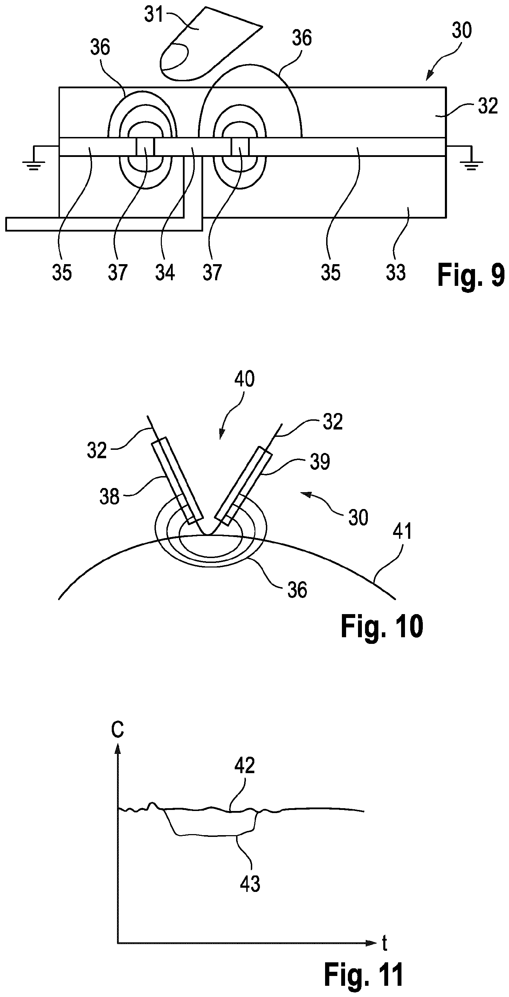

Als weitere Variante können kapazitive Sensoren in die Spitze des chirurgischen Instruments integriert sein. Die Figuren 8 und 9 zeigen schematisch die Wirkungsweise eines kapazitiven Sensors 30. Ein kapazitiver Sensor 30 umfasst zueinander abwechselnd angeordnete Elektroden 34 und 35, welche jeweils einen Abstand 37 zueinander aufweisen jeweils Platten eines Kondensators bilden. In der gezeigten Variante sind die Elektroden oder Platten 35 geerdet und die Elektrode 34 ist elektromagnetisch geladen. Die Elektroden 34 und 35 sind auf einem Substrat 33 angeordnet und werden von einer nicht-leitenden Schicht 32, beispielsweise aus Glas oder Plastik, abgedeckt.

Kapazitive Sensoren ermöglichen eine Messung des Kontakts oder annähernden Kontakts zu einem bestimmten Material oder einem biologischen Gewebe, z.B. einem Finger 31 einer Person. Kapazitive Sensoren werden vielfach als Eingabevorrichtungen für elektronische Geräte verwendet, wobei die Nähe eines Fingers 31 zu dem Sensor 30 das elektromagnetische Feld 36 des Sensors 30 verändert. Aus der Änderung des elektromagnetische Feldes 36 lässt sich der Abstand oder ein Kontakt ableiten auswerten. Dies ist in den Figuren 8 und 9 dargestellt, wobei durch eine Bewegung des Fingers 31 die elektromagnetischen Felder 36 verändert werden.

Das beschriebene Prinzip kann in einer einfachen Form im Rahmen eines chirurgischen Instruments, beispielsweise eines Cystotomes, angewendet werden. Dies ist in der Figur 10 schematisch gezeigt, wo mit einer Spitze 40 eines Cystotomes auf einen Kapselsack 41 eines Auges eingewirkt wird. Die scharfe Spitze 40 kann Elektroden 38 und 39 umfassen, zwischen welchen ein elektromagnetisches Feld 36 erzeugt werden kann und welche mit einer nicht-leitenden Schicht 32 bedeckt sein. Dabei kennzeichnen die Elektrode 38 ein Rx-Äquivalent, also eine Reciever-Elektrode, und die Elektrode 39 ein Tx-Äquivalent, also eine Transmitter-Elektrode. Für eine OVD-Umgebung (OVD - Ophthalmic Viscoelastic Device) ist eine angenommene Standardkapazität bekannt. In der Nähe des Kapselsacks 41 weicht die Kapazität hiervon ab und ermöglicht damit eine Abstands- und/oder Kontakt-Erfassung. Dies ist in der Figur 11 in Form eines Diagramms gezeigt, wobei auf der x-Achse die Zeit t und auf der y-Achse die Kapazität C jeweils in beliebigen Einheiten aufgetragen sind. Die Kurve 42 kennzeichnet die Ausgangskapazität vor einer Berührung, z.B. des Kapselsacks 41 durch die Spitze 40. Bei einer Berührung zwischen Spitze 40 und Kapselsacks 41 sinkt die Kapazität. Dies veranschaulicht die Kurve 43. Nach einer Berührung, also wenn die Spitze 40 von dem Kapselsack 41 entfernt wird, kehrt die Kapazität zu den Werten der Kurve 42, also zu dem Ausgangswert, zurück.

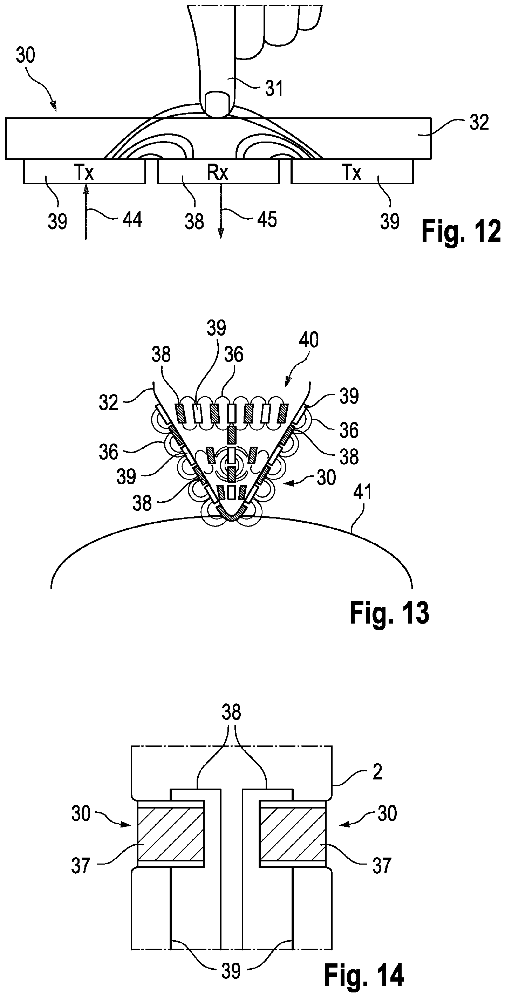

Die Elektroden 38 und 39 können in einen in eine leitfähige Schicht geätzt sein. Die Elektroden 38 und 39 können den in dem in der Figur 12 gezeigten Beispiel eines kapazitiven Sensors gezeigten Elektroden 38 und 39 entsprechen. In dem in der Figur 12 gezeigten Beispiel empfangen die Elektroden 39 ein mit der Bezugsziffer 44 gekennzeichnetes Eingangssignal und über die Elektrode 38 wird ein mit der Bezugsziffer 45 gekennzeichnetes Ausgangssignal zur Bestimmung der Kapazität erfasst.

Die Figur 13 zeigt eine Variante der in der Figur 10 gezeigten Variante, bei welcher in die Spitze 40 eine beliebige Vielzahl und Anordnung an kapazitiven Sensoren bestehend aus Elektroden 38 und 39 integriert sind. Die Elektroden 38 und 39 sind dabei jeweils abwechselnd zueinander angeordnet und umschließen die Spitze 40. Diese Variante hat den Vorteil, dass sie eine sehr genaue und räumlich präzise Erfassung eines Kontakts zwischen der Spitze 40 und dem Kapselsack 41 ermöglicht. Es kann insbesondere eine verbesserte Auflösung der Eindringtiefe der Spitze 40 in das Gewebe des Kapselsacks 41 erzielt werden und es können Informationen hinsichtlich der Ausrichtung der Spitze 40 bezüglich des Gewebes des Kapselsacks 41, insbesondere der Oberfläche des Kapselsacks 41, erfasst werden. Im Vergleich zu der in der Figur 10 gezeigten Variante ist der Herstellungsprozess allerdings aufwendiger und die Gesamtkosten sind höher. Die gezeigte Variante lässt sich selbstverständlich auch für beliebige andere chirurgische Instrumente anwenden.

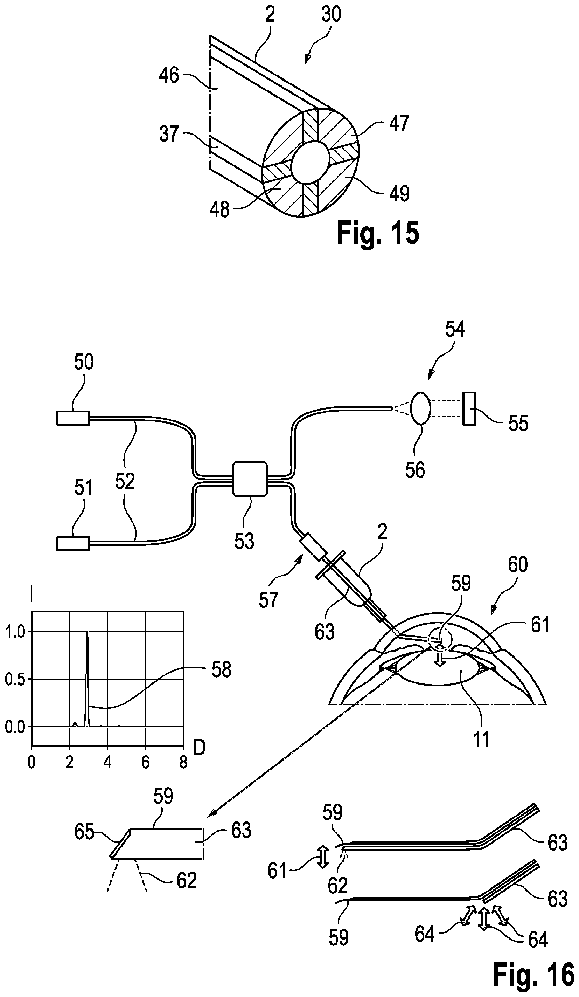

Die Figuren 14 und 15 zeigen weitere Varianten einer Integration eines kapazitiven Sensors 30 in ein chirurgisches Instrument 2. Die Figur 14 zeigt schematisch einen Teilbereich eines chirurgischen Instruments 2, welcher Vertiefungen aufweist, in denen die kapazitiven Sensoren 30 angeordnet sind, also die Elektroden 38 und 39 sowie ein zwischen den Elektroden 38 und 39 angeordnetes dielektrisches Material. In der gezeigten Variante sind die Elektroden 38 und die Elektroden 39 in axialer Richtung bzw. Längsrichtung des chirurgischen Instruments 2 hintereinander angeordnet. Bei einer Deformation oder Auslenkung des chirurgischen Instruments 2 in radialer Richtung verändern sich die Abstände zwischen den Elektroden 38 und 39. Hieraus lässt sich das Ausmaß der Deformation, beispielsweise in Form eines Abknickens oder Verbiegens, des chirurgischen Instruments 2 ableiten. Weiterhin können hieraus Kräfte ermittelt werden, welche auf das chirurgische Instrument 2 wirken oder von diesem auf ein Gewebe ausgeübt werden.

Wenn die Vertiefungen mit den kapazitiven Sensoren 30 in zwei Ebenen der Geometrie des chirurgischen Instruments 2 angeordnet werden, können Auslenkungen in zwei Dimensionen gemessen werden. Beispielsweise kann es sich bei der in der Figur 14 gezeigten Abbildung um eine Schnittansicht in einer ersten axialen Ebene handeln. Für eine zweidimensionale Messung kann in diesem Fall eine analoge Anordnung in einer hierzu senkrechten axialen Ebene vorgesehen sein.

Eine weitere Alternative ist in der Figur 15 gezeigt. Die Figur 15 zeigt schematisch einen Teilbereich eines chirurgischen Instruments 2 in einer quergeschnittenen und perspektivischen Ansicht. In diesem Fall umfasst das chirurgische Instrument 2 mindestens zwei, in der gezeigten Variante vier, zueinander symmetrisch angeordnete Stäbe mit einer festgelegten, vorzugsweise identischen, Querschnittsgeometrie. Die Stäbe 46, 47, 48 und 49 wirken als Elektroden und sind voneinander durch ein leicht elastisches dielektrisches Material 37 separiert. Wenn sich die Stäbe bzw. Elektroden 46, 47, 48, 49 verbiegen, sich beispielsweise leicht gegeneinander verschieben, wird eine Deformation des dielektrischen Materials 37 bewirkt. Dies hat eine Veränderung der Kapazität zur Folge und lässt sich als mindestens ein Kapazitätswert im Falle von zwei Stäben oder eine Mehrzahl an Kapazitätswerten im Falle von mehr als zwei Stäben auswerten und zur Deformations- und/oder Kraftbestimmung nutzen.

Im Folgenden wird anhand der Figur 16 ein in ein chirurgisches Instrument 2 integrierter Abstandssensor beschrieben. Oben rechts in der Figur 16 ist die optische Anordnung für eine Abstandsmessung mittels eines OCT-A-Scans gezeigt. Links ist ein Diagramm eines erfassten OCT-A-Scan Signals gezeigt. Unten sind drei vergrößerte Ansichten der Spitze 59 des chirurgischen Instruments 2 gezeigt. In der gezeigten Variante wird ein Auge 60 mittels eines chirurgischen Instruments 2 behandelt. Dabei kann beispielsweise der Kapselsacks oder eine Linse im Rahmen einer Katarakt-Operation behandelt werden. In das chirurgische Instrument 2 ist ein miniaturisierter Abstandssensor unmittelbar integriert. Bei dem chirurgischen Instrument 2 kann es sich zum Beispiel um eine Kapulotomie-Sonde, eine Cystotome-Nadel, eine Zange, ein Rhexis-Instrument oder ein anderes chirurgisches Instrument handeln. Das chirurgische Instrument 2 kann manuell oder robotisch führbar ausgestaltet sein.

Die Spitze des chirurgischen Instruments 2 ist mit der Bezugsziffer 59 gekennzeichnet. Der zu messende Abstand ist durch einen Pfeil 61 gekennzeichnet. Eine optische Faser 63, also ein lichtleitendes Bauteil, mit einer Optik in Form eines Aus- und Einkoppelelements 65, z.B. eines reflektiven optischen Elements, ist in das chirurgische Instrument 2 integriert. Die Sensorik kann dabei als OCT oder LIDA-Sensor ausgebildet sein. Mittels des Aus- und Einkoppelelements 65 werden Lichtstrahlen 62 in Richtung des zu behandelnden Gewebes ausgestrahlt und von dem Gewebe reflektiertes Licht empfangen. Die Lichtstrahlen werden von einer Lichtquelle 50 über optische Fasern 52 und einen Strahlteiler oder ein Interferometer 53 in die in das chirurgische Instrument 2 integrierte optische Faser 63 eingestrahlt. An der Spitze 59 in die optische Faser 63 eingekoppeltes Licht wird über den Strahlteiler oder das Interferonmeter 53 mittels einer optischen Faser 52 zu einem Detektor 51 geführt. Neben dem zu dem chirurgischen Instrument 2 führenden, als Probenarm 57 bezeichneten optischen Messpfad kann optional ein als Referenzarm 54 bezeichneter optischer Referenzpfad vorgesehen sein. Der Referenzarm 54 weist in der gezeigten Variante einen Spiegel 55 und eine Fokussieroptik 56 auf.

Eine Ablenkung des Lichts beim Durchgang durch die optische Faser 63 infolge einer durch die Pfeile 64 gekennzeichneten Veränderung der Krümmung des chirurgischen Instruments 2 lässt sich am distalen Ende der optischen Faser 63 bzw. 52 erfassen. Hierbei können zum Beispiel GRIN-Linsen (GRIN - Gradienten-Index) oder 3D-gedruckte Linsen oder winkelpolierte Fasern (ohne Gold-Beschichtung) oder Spiegel oder Kugellinsen zum Kollimieren, Fokussieren oder Ablenken des OCT-Strahls zur Verbesserung des Sensorsignals verwendet werden. Alternativ dazu kann die optische Faser 52, 63 durch Angle-Cleaving und Beschichtung zum Modifizieren des OCT-Strahls angepasst werden, um den Lichtstrahl in einem gewünschten Winkel in Richtung der Gewebeoberfläche abzustrahlen.

Im Falle einer Integration eines CT-A Abstandssensor in ein Cystrotome, zum Beispiel eine gebogene Nadel (siehe Figur 16), kann es erforderlich sein, den Lichtstrahl in einem bestimmten Winkel relativ zu der Faserachse abzulenken, um den Abstand zwischen der Spitze 59 und dem Gewebe zu messen. Die optische Faser 63 kann an verschiedenen Orten an dem Cystrotome angebracht sein. Für präzise Distanzmessungen muss ein Offset zwischen dem distalen Ende der Faser 63 und der Spitze 59 berücksichtigt werden, ebenso wie die gesamte geometrische Anordnung. Die erfassten Abstandsinformationen oder Tiefen-Abbildungen können auch dazu verwendet werden, um eine Wechselwirkung zwischen dem chirurgischen Instrument 2 und dem Gewebe 11 zu erfassen und auszuwerten, zum Beispiel um zu bestimmen an welchen Orten das Gewebe 11 bereits eingeschnitten oder behandelt worden ist. Zur Erfassung von Linsendeformationen oder einwirkenden Kräften können Rückstrahlungen aus Vorder- und Hinter-Oberflächen des Kapselsacks in Abhängigkeit von der Zeit ausgewertet werden.

In dem in der Figur 16 links gezeigten Diagramm ist die normierte Intensität I in Abhängigkeit von dem Abstand D der Spitze 59 des chirurgischen Instruments 2 von dem zu behandelnden Gewebe in Millimetern (mm) aufgetragen. Ein beispielhaft erfasstes OCT-A-Scan Signal ist mit der Bezugsziffer 58 gekennzeichnet. Die Verwendung von OCT-A-Scans bietet eine vielversprechende optische Technologie, welche eine miniaturisierte Abstandsmessung mit hoher Auflösung im Mikrometerbereich erlaubt, beispielsweise in Form von eindimensionalen Tiefen-Scans. Anstelle von OCT kann auch ein LIDAR-Sensor verwendet werden, welcher einfacher aufgebaut und damit unter Umständen kostengünstiger ist.

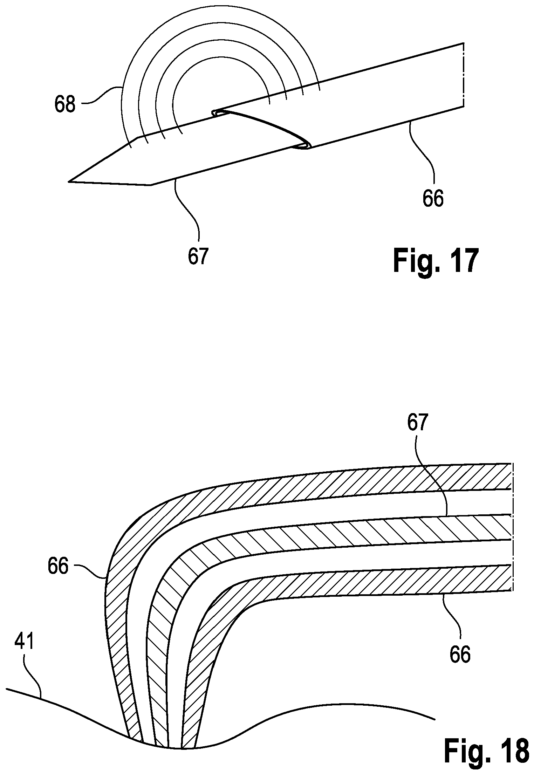

Im Folgenden werden anhand der Figuren 17 bis 20 in ein chirurgisches Instrument 2 integrierte Impedanz-Sensoren beschrieben. Gewebe kann mittels Hochfrequenzspannung, welche an ein chirurgisches Instrument angelegt wird, welches Kontakt mit dem Gewebe, hat unterschieden werden. Die übliche Frequenz liegt dabei oberhalb von 100 kHz, um Verletzungen für den Patienten zu vermeiden. Das Instrument kann zwei Elektroden (bipolare Anordnung) aufweisen, welche gegeneinander isoliert sind, oder eine Elektrode an dem Instrument und eine andere Elektrode an dem Körper des Patienten (monopolare Anordnung). Das Verfahren kann für Kapsulorhexis-Operationen zur Messung eines Kontakts eines chirurgischen Instruments mit dem Kapselsack angewendet werden. Hierzu ist eine Unterscheidung zwischen dem chirurgischen Instrument in dem OVD ohne Berührung des Kapselsacks und in dem OVD bei Berührung des Kapselsacks erforderlich. Für die bipolare Konfiguration kann das chirurgische Instrument aus zwei Nadeln bestehen, welche ineinandergeschoben angeordnet sind. Dies ist beispielhaft in den Figuren 17 und 18 gezeigt.

Die Figuren 17 und 18 zeigen ein in einer Nadelhülle 66 angeordnetes Stilett 67. Diese bilden die erforderlichen Elektroden. Das elektromagnetische Wechselfeld zwischen der Nadelhülle 66 und dem Stilett 67 ist mit der Bezugsziffer 68 gekennzeichnet. Beide Spitzen können, wie in der Figur 18 gezeigt, in dieselbe Richtung gebogen sein. Im Falle eines Kontakts mit dem Kapselsack 41 würde sich Kapselgewebe zwischen beiden Elektronen 66 und 67 befinden und eine Impedanzänderung bewirken, welche messbar wäre. Im Falle einer monopolaren Ausgestaltung ist eine einzelne Nadel ausreichend. Dabei kann es sich um ein übliches Cystotome handeln. Eine Isolation der äußeren Elektrode 66, also beispielsweise der Nadelhülle 66, gegenüber der Umgebung entlang des Schafts kann erforderlich sein, insbesondere um die Entstehung eines Stromkreises mit dem Hornhauteinschnitt zu vermeiden. Messungen bei verschiedenen Frequenzen können ebenso wie das Erfassen der Phase zwischen Strom und Spannung zusätzliche Informationen liefern.

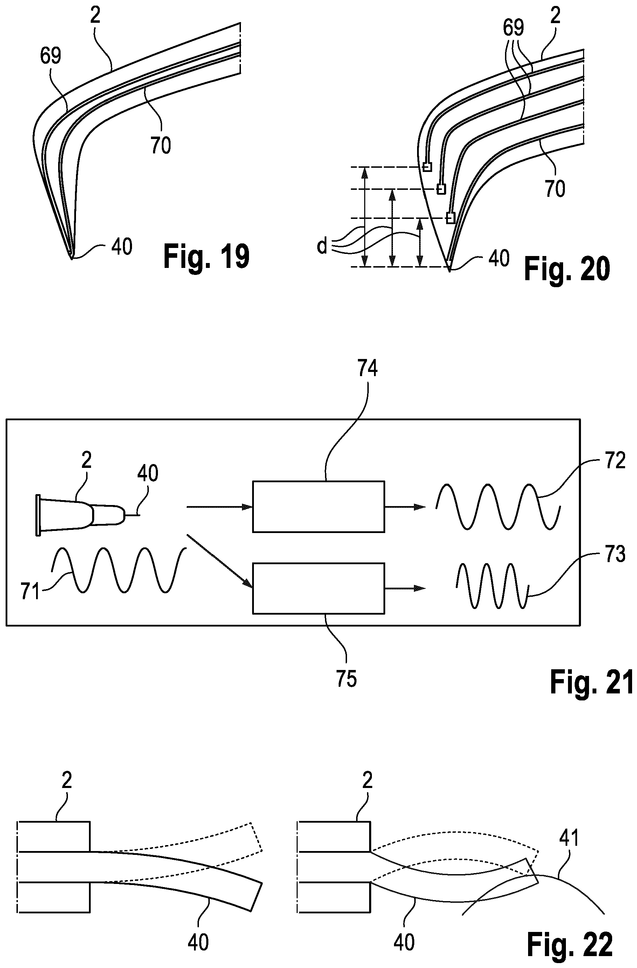

Prinzipiell können die die Elektroden bildenden leitfähigen Bereiche auch in das chirurgische Instrument 2 geätzt sein. In diesem Fall kann das chirurgische Instrument 2 eine nichtleitende Schicht und eine leitende Schicht aufweisen. Die leitende Schicht kann in der Weise geätzt werden, dass mindestens zwei separate und isolierte Elektroden erzeugt werden. Dies ist beispielhaft in der Figur 19 gezeigt. Eine erste Elektrode ist dabei mit der Bezugsziffer 69 gekennzeichnet und eine zweite Elektrode mit der Bezugsziffer 70. Das Prinzip kann auf weitere Elektroden ausgeweitet werden. Die Figur 20 zeigt hierfür ein Beispiel für ein Elektroden-Array, wobei eine Mehrzahl an ersten Elektroden 69 in verschiedenen Abständen oder Höhen d von der Spitze 40 des chirurgischen Instruments 2 angeordnet sind. Dies bietet die Möglichkeit einer Messung, wie tief das chirurgische Instrument 2 bereits in das Gewebe eingedrungen ist. Für ein bipolares Design kann die zweite Elektrode 70 als Spitze 40 des chirurgischen Instruments 2 ausgestaltet sein. Für ein monopolares Design ist keine zweite Elektrode 70 erforderlich.

Im Folgenden werden Sensormethoden zur Erfassung eines Kontakts basierend auf einer zusätzlichen Anregung des Sensors an einer Schnittstelle zwischen einem robotischen Arm und dem chirurgischen Instrument beschrieben. Bei einer oszillatorischen Anregung des chirurgischen Instruments kann eine resultierende Oszillation im Hinblick auf Phase und/oder Frequenz ausgewertet werden. Zum Beispiel kann ein Kontakt zwischen einem chirurgischen Instrument und einem Kapselsack durch Vibrationsanregung des chirurgischen Instruments mit einer hohen Frequenz und geringer Amplitude erfasst werden, beispielsweise mithilfe eines Piezo-Elements. Die Vibration kann entlang der Längsachse des chirurgischen Instruments oder lateralen oder entlang der Sichtachse erfolgen.

Das Prinzip ist in der Figur 21 dargestellt. Die Spitze 40 des chirurgischen Instruments 2 vibriert mit einer Anregungsfrequenz 71. In dem Fall, dass kein Kontakt zu einem Gewebe besteht, dargestellt durch Kasten 74, entspricht die Antwortfrequenz 72 der Anregungsfrequenz 71. In dem Falle, dass die Spitze 40 in Kontakt mit einem Gewebe kommt, dargestellt durch Kasten 75, weicht die Antwortfrequenz 73 von der Anregungsfrequenz 71 ab. Aus der Veränderung der Antwortfrequenz 73 im Vergleich zur Anregungsfrequenz 71 lassen sich Rückschlüsse auf den Kontakt, beispielsweise den Abstand oder die Eindringtiefe oder die wirkende Kraft ziehen.

Im freien Raum oder im OVD würde die Anregung zu einer Vibration der Spitze 40, wie in der Figur 22 links gezeigt, führen. Im Falle eines Kontakts der Spitze 40 mit einem Gewebe, zum Beispiel einem Kapselsack 41, würde die Vibration gedämpft oder es würde ein Knotenpunkt entstehen. Dies ist in der Figur 22 rechts gezeigt. Die gedämpfte Amplitude kann den Kontakt zwischen dem chirurgischen Instrument 2 und dem Gewebe 41 abbilden und gegebenenfalls Informationen über eine Eindringtiefe des chirurgischen Instruments 2 in das Gewebe 41 liefern.

Zur Messung der Vibrationen können in die Oberfläche des chirurgischen Instruments 2 Dehnungsstreifen, wie beispielsweise in den Figuren 6 und 7 gezeigt, integriert sein oder diese können in einem Befestigungseinrichtung 7 (siehe Figur 2) für das chirurgische Instrument 2 an einer Schnittstelle zwischen einem robotischen Manipulator 6 und dem chirurgischen Instrument 2 angeordnet sein.

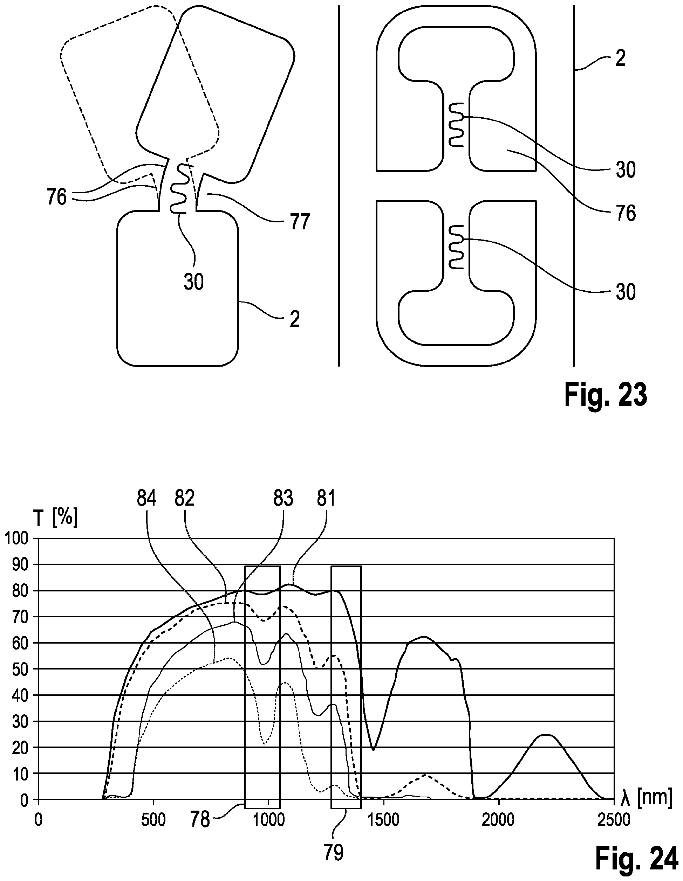

Alternativ dazu kann, wie in der Figur 23 rechts gezeigt, ein Verbindungselement 76 vorgesehen sein, welches zu einer stärkeren Vibration des distalen Teils des chirurgischen Instruments 2 führt. Hierdurch können Einflüsse des Kontakts auf einen Hornhauteinschnitt vermieden werden. Eine andere Option besteht darin, Einschnitte oder Ausschnitte 77 in der Struktur des chirurgischen Instruments 2, wie in der Figur 23 links gezeigt, vorzusehen. Die Ausschnitte 77 sind dergestalt, dass sie auf Anregungsfrequenzen reagieren, welche höhere Amplituden als der gesamte Körper des chirurgischen Instruments 2 aufweisen. Lokal integrierte Dehnungsstreifen 30 können zur Messung der Vibrationen verwendet werden. Eine Messung der Vibrationen kann auch optisch unter Verwendung von beispielsweise einem Laser-Doppler-Vibrometer erfolgen.

Weiterhin können aufgrund der Vibrationen auftretende Deformationen mittels kapazitiver Sensoren, beispielsweise wie in den Figuren 14 und 15 gezeigt, erfasst werden. Die wechselnden Abstände zwischen den Elektroden aufgrund der Verformungen führen zu einer sich verändernden Kapazität, mittels welcher die Verformungen bestimmt werden können. Der Hersteller SURAC verwendet auditive bzw. akustische Feedbacks von dem chirurgischen Instrument zur Auswertung von Gewebeinteraktionen. Ein ähnliches Konzept kann in Kombination mit einem robotischen System verwendet werden.

In einer weiteren Variante können mehrachsige Kraft- und/oder DrehmomentSensoren an einem Manipulator 6 befestigt sein und zur Messung der an dem chirurgischen Instrument 2 angreifenden Kräfte verwendet werden. Typischerweise ist das chirurgische Instrument an zwei Punkten mit dem Auge im Kontakt. Dabei handelt es sich einmal um den Eintrittspunkt in das Auge, beispielsweise einen Hornhautschnitt oder einen Trokar. Ein zweiter Kontaktpunkt besteht zwischen der Spitze des chirurgischen Instruments und dem Augengewebe. Beide Kontaktpunkte führen zu Kräften und Drehmomenten, welche mittels des Kraft- und/oder Drehmoment-Sensors messbar sind.

Für eine bekannte relative Position zwischen dem Kraft- und/oder Drehmoment-Sensor und dem Eintrittspunkt in das Auge kann ein Modell erstellt werden, welches eine Berechnung der Kräfte der Spitze des chirurgischen Instruments aus den Sensorausgaben des Kraft- und/oder Drehmomentsensors ermöglicht. Der Eintritts- oder Einschnittpunkt in das Auge kann detektiert und/oder nachverfolgt werden, beispielsweise mittels eines lateralen Sensorsystems oder einem zusätzlichen Sensorsystem. Stand der Technik hierzu ist in Schäfer et al., Robotic Telemanipulation System for Minimally Invasive Surgery using a Passive Universal Joint and Internal Sensors, Hamlyn Symposium 2022 offenbart.

Im Folgenden werden Beispiele für eine Erfassung eines Kontakts zwischen einem Gewebe und einem chirurgischen Instrument durch eine Beobachtung von Gewebereaktionen beschrieben. Eine erste Variante besteht darin, optische Reflektionen durch das Gewebe bei einer Beleuchtung, beispielsweise mit einem Muster, zu erfassen und auszuwerten. Hierbei können insbesondere Wellenlängen aus dem nicht sichtbaren Spektrum verwendet werden.

Die Figur 24 zeigt schematisch die Transmission von Licht in Abhängigkeit von der Wellenlänge durch verschiedene Gewebe eines Auges in Form eines Diagramms. Auf der x-Achse ist die Wellenlänge λ des verwendeten Lichts in Nanometern (nm) aufgetragen. Auf der y-Achse ist die Transmission T in Prozent angegeben. Die Kurve 81 zeigt das Transmissionsverhalten der Hornhaut eines Auges. Die Kurve 82 zeigt das Transmissionsverhalten einer mit Wasser bedeckten Hornhaut, also einer wässrigen Hornhaut. Die Kurve 83 zeigt das Transmissionsverhalten von Hornhaut, Wasser und Linse zusammen. Die Kurve 84 zeigt das Transmissionsverhalten von Hornhaut, Waser, Linse und Glaskörper gemeinsam.

Während einer Katarakt-Operation wird Gewebe behandelt, welches im sichtbaren Wellenlängenbereich transparent ist. Kameras, welche meistens zur Nachahmung eines natürlichen Anblickes optimiert sind, können nur eine sehr begrenzte Anzahl an Merkmalen von transparentem Gewebe detektieren. Um eine größere Anzahl an Merkmalen z.B. der Linse und des Kapselsacks detektieren zu können, ist eine Wellenlänge besser geeignet, welche von dem Gewebe absorbierbar ist. Zur Erfassung von durch die Hornhaut gestrahltem Licht darf die jeweilige Wellenlänge durch die Hornhaut nicht zu stark absorbiert werden.

Mit der Verwendung von Infrarotlicht im Wellenlängenbereich von 800 nm bis 1800 nm, vorzugsweise einem schmalen Band in diesem Bereich - z.B. wie in der Figur 24 durch die Bereiche 78 und 79 gekennzeichnet -, kann die Linse zwar erreicht, allerdings nicht signifikant durchstrahlt werden. Die in der Figur 24 mit den Bezugsziffern 78 und 79 gekennzeichneten Wellenlängenbereiche stellen bevorzugte Ausführungsvarianten dar. Entsprechend angepasste Kameras, zum Beispiel Silizium-basiert für bis zu 1100 nm oder InGaAsbasiert für einen Wellenlängenbereich zwischen 400 nm und 1800 nm, bieten daher einen besseren Kontrast für Merkmale des menschlichen Auges und ermöglichen es mittels Bildverarbeitungsverfahren die dreidimensionale Form der Linse als Stereoabbildung zu rekonstruieren.

Zusätzlich können diese Wellenlängen genutzt werden, um Schatten von dem chirurgischen Instrument auf den Kapselsack zu projizieren. Die Bildinformation des Schattens kann zum Abschätzen des Abstandes zwischen dem chirurgischen Instrument und dem Gewebe genutzt werden. Das Infrarotbild kann auf einen Bildschirm übertragen werden, wo das Bild mit falschen Farben rekonstruiert und einem Operateur bzw. medizinischem Personal angezeigt wird.

In einer weiteren Variante kann das beschriebene Verfahren mit einer strukturierten Beleuchtung zur Abbildung von Mustern auf dem jeweiligen Gewebe, zum Beispiel der Linse oder dem Kapselsack, und zur Auswertung der Muster verwendet werden. Diese Muster können als zusätzliche Merkmale durch das Sensorsystem detektiert werden. Strukturierte Beleuchtung stellt eine Technik dar, die es ermöglicht, anhand des auf die Oberfläche projizierten Lichtmusters Oberflächenformen und Tiefeninformationen zu berechnen.

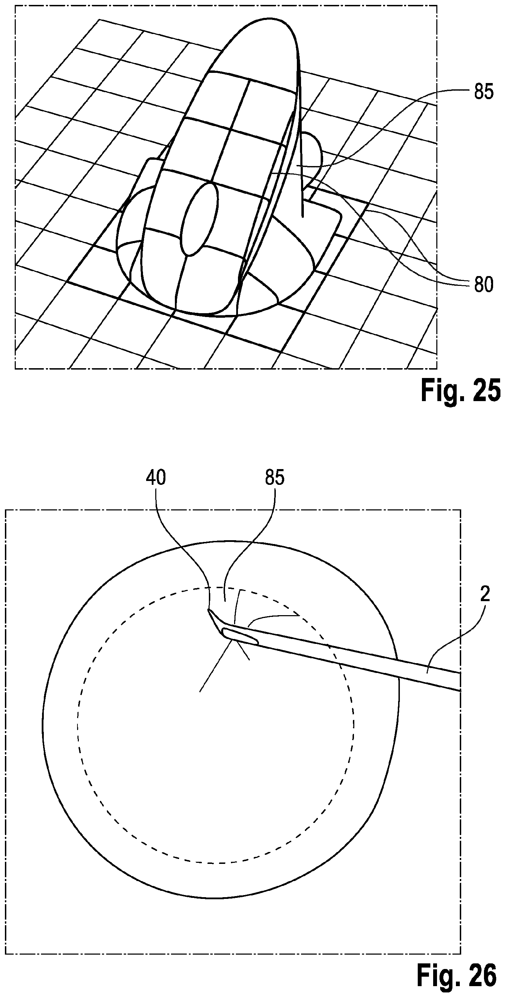

Die Beleuchtungsquelle kann entweder in ein Mikroskop oder ein Werkzeug, zum Beispiel ein chirurgisches Instrument, integriert sein. Das Erfassen von Informationen kann entweder mit einer oder mehreren Kameras erfolgen. Da sichtbares Licht auf transparentem Gewebe eines Auges kein klar sichtbares Muster erzeugt, vereinfacht die Verwendung von Infrarotlicht die Detektion von zu scanbaren Mustern, zum Beispiel auf der Linsenoberfläche. Zur Abbildung der Retina wurde die Verwendung von strukturierter Beleuchtung zum Beispiel in S. Gruppetta, "Structured Illumination for In-Vivo Retinal Imaging," in Frontiers in Optics 2013, I. Kang, D. Reitze, N. Alic, and D. Hagan, eds., OSA Technical Digest (online) (Optica Publishing Group, 2013), paper FW2F.1. (https://opg.optica.org/abstract.cfm?URI=FiO-2013-FW2F.1) vorgeschlagen. Ein Beispiel für ein Bild eines mit strukturierter Beleuchtung beleuchteten Gewebes 85 ist in der Figur 25 gezeigt. Das erzeugte Muster ist vorliegend ein quadratisches Gittermuster 80. Aus diesem lässt sich die dreidimensionale Form des Gewebes rekonstruieren.

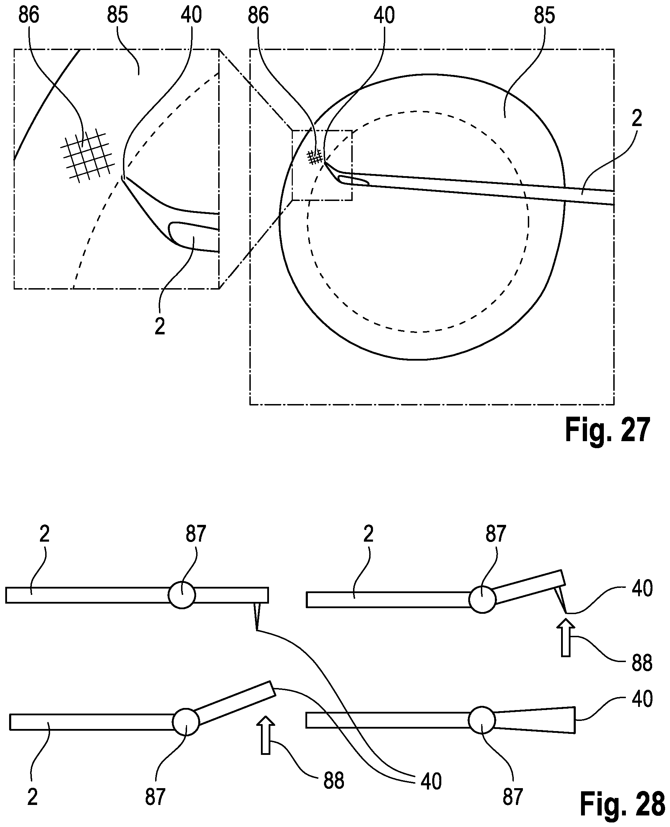

Eine weitere Möglichkeit zur Erfassung eines Kontakts zwischen einem chirurgischen Instrument und einem Gewebe oder zur Erfassung des Drucks oder der Kraft, die ein chirurgisches Instrument auf ein Gewebe ausübt, besteht darin, die Veränderungen der Helligkeit des von dem Gewebe reflektierten Lichts in unmittelbarer Umgebung des Bereichs des Gewebes, in welchen eine Einwirkung stattfindet, zum Beispiel in der Umgebung einer Spitze des chirurgischen Instruments, zu erfassen und auszuwerten. Ein Beispiel hierfür ist in den Figuren 26 und 27 gezeigt. In der Figur 26 ist ein chirurgisches Instrument 2 mit seiner Spitze 40 über einem Gewebe 85 eines Auges angeordnet. Es findet noch kein unmittelbarer Kontakt zwischen der Spitze 40 des chirurgischen Instruments 2 und dem Gewebe 85 statt. Es ist daher in der Umgebung der Spitze 40 auf dem Gewebe 85 auch kein Schatten zu beobachten. Im Unterschied dazu ist in der in der Figur 27 gezeigten Variante in der Umgebung der Spitze 40 ein Schatten zu beobachten, da in dieser Variante die Spitze 40 das Gewebe 85 berührt und einen Druck auf dieses ausübt.

Mit einer typischen Beleuchtung und Lichtdetektion in Operationsmikroskopen gemäß dem Stand der Technik sind bei einer Einwirkung auf eine flache Oberfläche eines Gewebes, zum Beispiel beim Drücken mit einem chirurgischen Instrument auf die Oberfläche eines Gewebes, wie zum Beispiel auf den Kapselsack, um die Spitze des chirurgischen Instruments herum an dem Interaktionspunkt Schatten zu beobachten, wie beispielsweise in der Figur 27 gezeigt. Bildverarbeitungsverfahren können zum Detektieren solcher Veränderungen des Bildes bei einer Interaktion zwischen chirurgischem Instrument und Gewebe verwendet werden. Die auf diese Weise gewonnenen Informationen können dazu genutzt werden, um zu entscheiden, ob eine abgestrebte Eindringtiefe des chirurgischen Instruments in das Gewebe erreicht ist und oder ob ein angestrebter Druck auf das Gewebe ausgeübt wird. Der Kontrast des Schattens kann mittels geeigneter Techniken erhöht werden, zum Beispiel durch Verwendung bestimmter Wellenlängen oder polarisationssensitiver Abbildungen durch differenziellen Interferenzkontrast (DIC - differential interference contrast).

Im Folgenden werden Beispiele beschrieben, in welchem ein Kontakt zwischen einem chirurgischen Instrument und einem Gewebe erfasst wird, indem infolge der Einwirkung auf das Gewebe erzeugte mechanische Deformationen des chirurgischen Instruments in optisch erfassbare Effekte transformiert und mit einem lateralen Sensorsystem ausgelesen werden. Dabei kann insbesondere eine herabgesetzte Steifheit des chirurgischen Instruments vorgesehen sein und infolgedessen eine Deformation des chirurgischen Instruments bei einer Einwirkung auf ein Gewebe makroskopisch beobachtbar sein.

Üblicherweise verwendete Cystotome sind so steif, dass Deformationen während einer gewöhnlichen Kapsulorhexis-Operation kaum auftreten. Um in der Lage zu sein, mittels zum Beispiel eines ophthalmischen Mikroskops, Verformungen oder Auslenkungen infolge von typischen Kräften während einer Kapsulorhexis-Operation visuell zu erfassen, kann das chirurgische Instrument im Hinblick auf seine Steifheit angepasst sein, insbesondere so dass die Verformungen vergrößert werden und mit einer geeigneten Auflösung deutlich erkennbar sind. Die Größenordnung hängt hierbei unter anderem von dem verwendeten optischen Sensor ab. Ein Ausgangs-Ansatz kann es sein, ein Cystotome mit verringerter Außenwanddicke zu verwenden, was die gesamte Steifheit des Werkzeugs reduziert. Dies führt dazu, dass sich das Werkzeug bei einer Krafteinwirkung über seine gesamte Länge verbiegt.

In eine Alternative dazu können bestimmte Bereiche des chirurgischen Instruments zum Beispiel durch Einbringen von Vertiefungen in dessen Geometrie geschwächt ausgestaltet sein. Das Einbringen von Vertiefungen kann zum Beispiel mittels Mikrofräsen oder Ätzen oder Laserschneiden erfolgen. Der geschwächte Bereich fungiert dann als Gelenk und bewirkt eine lokale Verformung an dem Gelenk im Falle einer Krafteinwirkung. In einer weiteren Variante kann das chirurgische Instrument anstelle einer zylindrischen Form als Streifen ausgestaltet sein. Die zuvor beschriebenen Ausgestaltungsvarianten verbessern die laterale Verformung des chirurgischen Instruments.

Für bestimmte Anwendungen kann auch eine vertikale Verformung, also eine Verformung in Richtung der Sichtachse oder optischen Achse des Mikroskops, von Bedeutung sein. Im Falle von vertikalen Verformungen bewirkt eine reduzierte Steifheit des chirurgischen Instruments keine verbesserte Visualisierbarkeit, da die Kamera das chirurgische Instrument von oben erfasst und die Deformationen in Richtung der Sichtachse oder optischen Achse der Kamera auftreten würden.

Die Figur 28 zeigt schematisch ein Beispiel für ein chirurgisches Instrument, welches mit einem geschwächten Bereich oder einem Gelenk 87 ausgestaltet ist. Dabei ist das chirurgische Instrument 2 in den beiden oben gezeigten Varianten in einer Seitenansicht und in den beiden unten gezeigten Varianten in einer Draufsicht gezeigt. Links ist die Einwirkung einer lateralen Kraft und rechts die Einwirkung einer normalen Kraft, also einer senkrecht zur Gewebeoberfläche wirkenden Kraft, gezeigt. Die Richtung der Krafteinwirkung ist jeweils durch Pfeile mit der Bezugsziffer 88 gekennzeichnet. In der links gezeigten Variante wird bei Einwirkung einer lateralen Kraft die Spitze des chirurgischen Instruments seitlich ausgelenkt. In der rechts gezeigten Variante wird bei Einwirkung einer normalen Kraft die Spitze des 40 des chirurgischen Instruments 2 nach oben ausgelenkt.

Die bisher beschriebenen Ausgestaltungsvarianten verbessern die laterale Verformbarkeit des chirurgischen Instruments. Für Anwendungen sind aber auch vertikale oder axiale Verformungen von Bedeutung. Für axiale Verformungen bewirkt eine reduzierte Steifheit bzw. ein entsprechender geschwächter Bereich oder ein entsprechendes Gelenk eine Biegung oder Verformung des chirurgischen Instruments wie oben rechts in der Figur 28 gezeigt. Die Kameras beobachten das chirurgische Instrument üblicherweise von oben und die Deformation würde in Richtung der Sichtachse oder der optischen Achse erfolgen und damit schwer erfassbar sein.

Wenn die Position, insbesondere die gewünschte Position, des chirurgischen Instruments basierend auf einem kinematischen Modell berechnet werden kann und die aktuelle Position der Spitze des deformierten chirurgischen Instruments im Hinblick auf ihre Tiefe mittels einer Stereokamera gemessen werden kann, so kann hieraus die Verformung basierend auf den Positionen des gewünschten Positionspunktes und des aktuellen Positionspunktes abgeleitet werden. Hieraus lässt sich die einwirkende Kraft bestimmen. Die beschriebene Berechnung mittels eines kinematischen Modells setzt voraus, dass das chirurgische Instrument robotisch geführt wird, die Geometrie des chirurgischen Instruments bekannt ist und dieses in Bezug auf seine Position kalibriert ist.

Die obere Oberfläche des chirurgischen Instruments kann bestimmte reflektive Merkmale aufweisen, welche Licht in einem bestimmten Winkel reflektieren, wenn das chirurgische Instrument deformiert ist. Entsprechende Merkmale können zum Beispiel sphärische Vertiefungen sein. Auch führen Reflexionen von einem zylindrischen Schaft eines chirurgischen Instruments zu einem spezifischen Reflexionsmuster, welches sich infolge einer Deformation verändert. Hieraus lässt sich die Deformation und damit auf das Gewebe ausgeübte Kräfte ableiten.

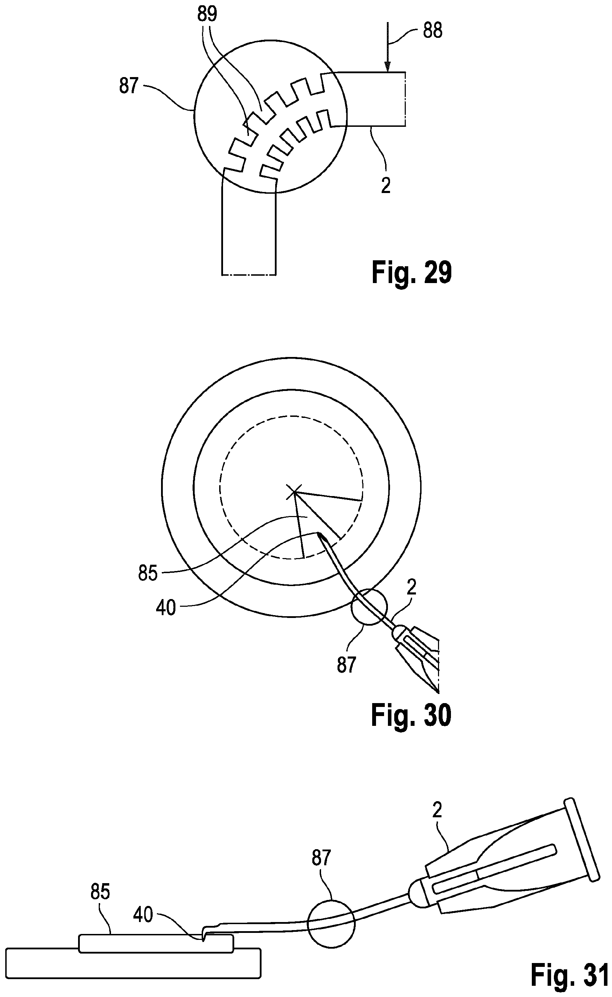

Die Figur 29 zeigt schematisch einen Teilbereich eines chirurgischen Instruments 2 mit einem geschwächten Bereich 87. Die Oberfläche des chirurgischen Instruments weist in dem Bereich 87 Vertiefungen 89 auf, welche eine erhöhte Deformierbarkeit des Bereichs 87 bewirkt. **In** der Figur 29 ist eine entsprechende Deformation des chirurgischen Instruments 2 infolge einer Krafteinwirkung 88 gezeigt.

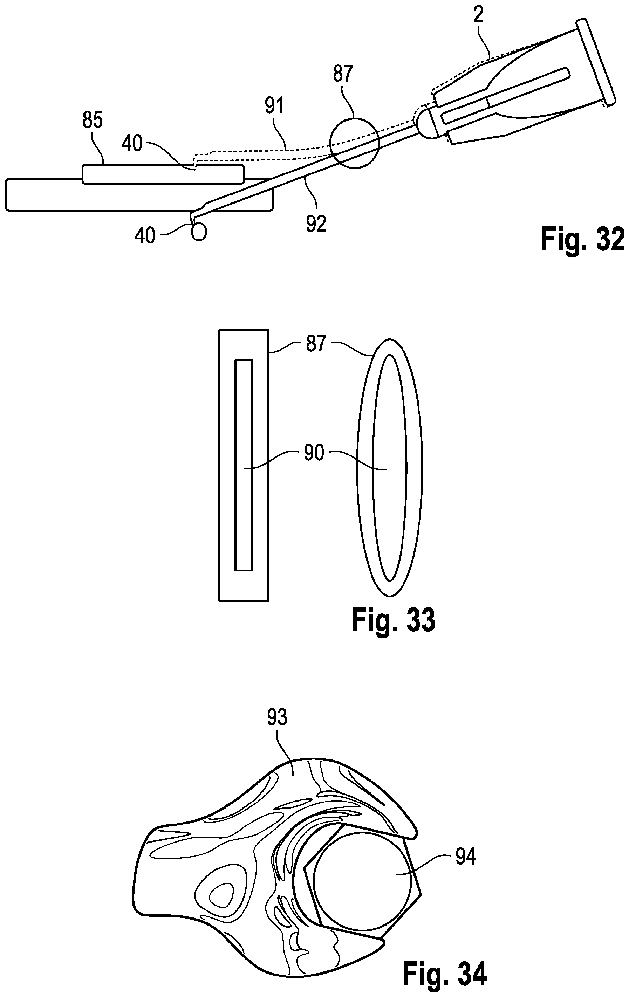

Die Figuren 30 zeigt ein Kapselgewebe eines Auges und ein auf dieses einwirkendes chirurgisches Instrument 2 in einer Draufsicht. **In** der Figur 31 ist eine entsprechende Seitenansicht gezeigt. Infolge der Einwirkung der Spitze 40 des chirurgischen Instruments 2 auf das Gewebe 85 entsteht in dem Bereich 87 eine detektierbare Deformation. Die Figur 32 zeigt schematisch in einer Seitenansicht einen Vergleich zwischen dem chirurgischen Instrument 2 in einem deformierten, also belasteten, Zustand 91 und einem nicht deformierten, also unbelasteten, Zustand 92. Mittels eines kinematischen Modells lässt sich aus der Abweichung des belasteten Zustandes 91 von dem unbelasteten Zustand 92 die aktuelle Position der Spitze 40 des chirurgischen Instruments 2 im belasteten Zustand 91 berechnen. Hieraus kann die Eindringtiefe der Spitze 40 in das Gewebe 85 bestimmt werden.

Die Figur 33 zeigt eine geometrische Ausgestaltungsvariante des deformierbaren Bereichs 87 eines chirurgischen Instruments 2 in einer Draufsicht. Links ist ein nicht deformierter, also unbelasteter Zustand gezeigt, also ein Zustand ohne Krafteinwirkung. Rechts ist ein deformierter, also belasteter Zustand gezeigt, also ein Zustand bei Krafteinwirkung. Der Spalt oder die Einkerbung 90, welche/r in der gezeigten Geometrie des deformierbaren Bereichs 87 vorgesehen ist, verbreitert sich im Falle einer durch Krafteinwirkung auftretenden Deformation. Dies ist in der Figur 33 rechts zu sehen. Der gezeigte Effekt ist visuell sehr gut detektierbar, zum Beispiel in einer mikroskopischen Ansicht.

Eine weitere Variante zur Bestimmung der von einem chirurgischen Instrument ausgeübten Kraft besteht in der Nutzung von polarimetrischen Veränderungen des verwendeten Materials bei Krafteinwirkung. Werkzeuge, deren Material auf Plastik oder Polymer basiert, weisen doppelbrechende Merkmale auf, wenn sie in einer bestimmten Art und Weise behandelt werden. Die doppelbrechenden Effekte treten in Form von einzigartigen Bildern beim Erfassen mittels einer Polarisationskamera auf. Der doppelbrechende Effekt verändert sich in Abhängigkeit von der Belastung des Materials. Wenn ein entsprechendes Plastik-Werkzeug auf ein Gewebe gedrückt wird, führt die Belastung in dem Werkzeug zu einer Veränderung der Doppelbrechung des Materials. Dies kann mittels einer Polarisationskamera erfasst werden.

Wird beispielsweise ein polymerbasiertes Cystotome und ein Mikroskop, welches Polarisationsbilder erfassen kann, also eine polarisierte Beleuchtung und einen polarisationssensitiven Kamera-Chip aufweist, so kann die Stärke der Interaktion zwischen dem chirurgischen Instrument, z.B. dem Cystotome, und dem Gewebe während einer ophthalmischen Operation detektiert werden. Es kann also insbesondere die Krafteinwirkung des chirurgischen Instruments, zum Beispiel des Cystotomes, auf das Gewebe bestimmt werden. Die Figur 34 zeigt schematisch das Prinzip einer polarimetrischen Bestimmung der bei Krafteinwirkung in einem Werkzeug auftretenden Spannung. **In** der Figur 34 ist ein mittels einer Polarisation, war abgebildeter Schraubenschlüssel 93 gezeigt, welcher auf einen Schraubenkopf 94 ein Drehmoment ausübt. Die Krafteinwirkung auf den Schraubenschlüssel 93 führt zu einer Spannung in dem Material des Schraubenschlüssels. Hierdurch werden die doppelbrechenden Eigenschaften des Materials lokal verändert und können mittels einer Polarisationskamera sichtbar gemacht werden und anschließend ausgewertet werden.

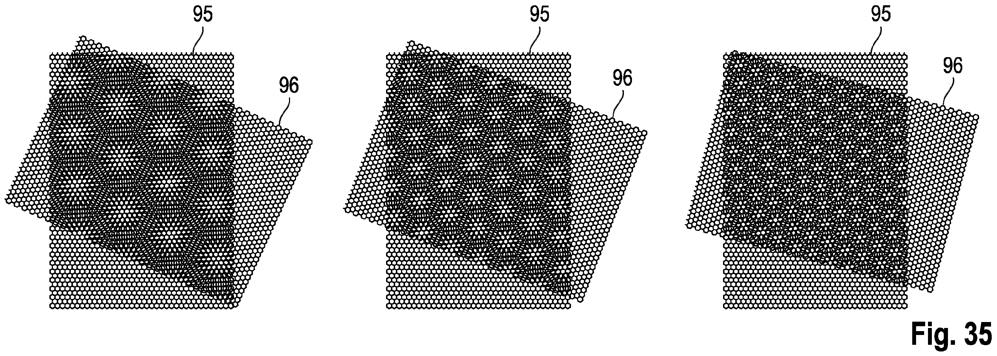

Eine weitere Variante zum Erfassen einer Deformation des chirurgischen Instruments während einer Einwirkung auf ein Gewebe besteht in der Nutzung von Moiré-Mustern oder der Verwendung der Vernier-Skala. Die Figur 35 zeigt schematisch Moiré-Muster, die bei aufeinander angeordneten, teilweise transparenten Mustern, beispielsweise Schachbrettmustern, abhängig von der relativen Position der beiden Muster zueinander entstehen. **In** dem gezeigten Beispiel sind jeweils ein erstes Bauelement 95 mit einem teilweise transparenten Muster und ein zweites identisches Bauelement 96 aufeinander angeordnet. Bei einer Verschiebung oder Drehung des Bauelement 96 in Bezug auf das Bauelement 95 entstehen in Abhängigkeit von der Positionsänderung erfassbare spezifische Muster. Diese können ausgewertet und zur Bestimmung der relativen Position der Bauelemente 95 und 96 zueinander verwendet werden.

Zur Anwendung dieses Prinzips im Zusammenhang mit einem chirurgischen Instrument kann das chirurgische Instrument zwei Tuben um oder Röhren fassen. Der innere Tubus kann ein eingraviertes, beispielsweise mittels eines Lasers eingraviertes, schachbrettartiges Muster aufweisen. Der äußere Tubus kann eine Hülse, z.B. eine Plastik-Hülse, mit demselben Schachbrettmuster sein, wobei die weißen Quadrate transparent ausgestaltet sein können oder ausgeschnitten sein können. Beide Tuben sind vorzugsweise nur an einem definierten Bereich, z.B. nur an ihrer Grundfläche oder einer festgelegten Kante, miteinander verbunden. In einer Ausgangsposition können die Schachbrettmuster einander perfekt überlappen. Bei einer Deformation entstehen aufgrund der Verschiebung der Schachbrettmuster zueinander neue spezifische Moiré-Muster, siehe Figur 35. Aus der Abhängigkeit der Moiré-Muster von der relativen Position der Tuben zueinander lässt sich das Ausmaß der Deformation, zum Beispiel einer Biegung oder Krümmung, des chirurgischen Instruments ableiten.

Die Verwendung von Moiré-Mustern hat den Vorteil, dass kleine strukturelle Deformationen bereits zu abweichenden Moiré-Mustern führen, welche mit einem Kamerasystem einfach erfasst werden können, insbesondere da das entstehende Moiré-Muster deutlich größere Abmessungen verglichen mit dem Ausmaß der Deformation aufweist. Es kann also die von einem chirurgischen Instrument auf ein Gewebe ausgeübte Kraft unter Verwendung von Moiré-Mustern, welche mittels eines Kamerasystems erfasst werden, bestimmt werden.

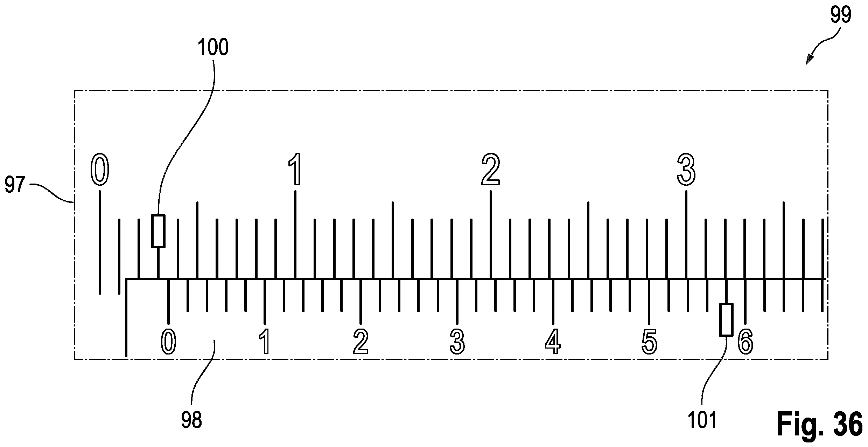

Eine Alternative zur Verwendung von Moiré-Mustern stellt die Nutzung der schematisch in der Figur 36 gezeigten Vernier-Skala, wie sie insbesondere von Messschiebern bekannt ist, dar. Die Figur 36 zeigt schematisch einen Teilbereich eines Messschiebers zur Veranschaulichung der Vernier-Skala. Der Messschieber 99 weist eine erste Schiene 97 mit einer ersten Skala und eine dazu verschiebbar angeordnete zweite Schiene 98 mit einer zweiten Skala. Die Markierungen 100 und 101 kennzeichnen die Ablesepositionen für die gezeigte Einstellung. Mit der gezeigten Skala lässt sich eine Ablesegenauigkeit von 0,02 mm realisieren.

In einer Ausführungsvariante kann das chirurgische Instrument zwei verschiedene, im Falle einer Deformation zueinander verschiebbare Segmente, analog zu den in der Figur 36 gezeigten Schienen 97 und 98, mit einer Vernier-Skala aufweisen. Bereits geringfügige Verschiebungen der Segmente zueinander während einer Deformation des chirurgischen Instruments können unter Verwendung der Vernier-Skala optisch sichtbar und ablesbar gemacht werden.

### Bezugszeichenliste:

- 1: Steuervorrichtung
- 2: chirurgisches Instrument
- 3: Sensoreinrichtung
- 4: Einrichtung zum Erfassen lateraler Positionsdaten
- 5: Einrichtung zum Erfassen von Daten zu einer axialen Beziehung zwischen chirurgischem Instrument und Gewebeoberfläche
- 6: robotischer Manipulator
- 7: Befestigungseinrichtung
- 8: Steuerung / Signalübertragung
- 9: robotische Anordnung
- 10: Operationsmikroskop
- 11: Gewebe
- 12: Gewebeoberfläche
- 13: Mensch-Maschine-Schnittstelle
- 14: Faser-Bragg-Gitter-Sensor
- 15: optische Faser
- 16: optische Gitter
- 17: eingestrahltes Frequenzspektrum
- 18: reflektiertes Frequenzspektrum
- 19: transmittiertes Frequenzspektrum
- 20: Spitze
- 21: optische Faser
- 22: fester Bereich
- 23: Basisbereich
- 24: Verbindungsbereich
- 25: reflektive Oberfläche
- 26: eingestrahltes Licht
- 27: reflektiertes Licht
- 28: einzelne Faser
- 29: Hohlraum
- 30: kapazitiver Sensor
- 31: Finger
- 32: Deckplatte
- 33: Substrat
- 34: Elektrode
- 35: Elektrode
- 36: elektromagnetisches Feld
- 37: Abstand / Dielektrikum
- 38: Elektrode
- 39: Elektrode
- 40: Spitze eines chirurgischen Instruments
- 41: Kapselsack
- 42: Ausgangskapazität ohne Berührung
- 43: reduzierte Kapazität während Berührung
- 44: Eingangssignal
- 45: Ausgangssignal
- 46: Elektrode
- 47: Elektrode
- 48: Elektrode
- 49: Elektrode
- 50: Lichtquelle
- 51: Detektor
- 52: optische Faser
- 53: Interferometer, Strahlteiler
- 54: Referenzarm
- 55: Spiegel
- 56: Fokussieroptik
- 57: Messarm
- 58: OCT-A-Scan Signal
- 59: Spitze des chirurgischen Instruments
- 60: Auge
- 61: Abstandsmessung
- 62: Lichtstrahl
- 63: integrierte optische Faser
- 64: Bewegungsrichtungen
- 65: Ein- und Auskoppelelement
- 66: Nadelhülle
- 67: Stilett
- 68: elektromagnetisches Wechselfeld
- 69: erste Elektrode
- 70: zweite Elektrode
- 71: Anregungsfrequenz
- 72: Antwortfrequenz
- 73: Antwortfrequenz
- 74: kein Kontakt mit Gewebe
- 75: Kontakt mit Gewebe
- 76: Verbindungselement
- 77: Ausschnitt
- 78: Wellenlängenbereich
- 79: Wellenlängenbereich
- 80: mit strukturierter Beleuchtung erzeugtes Muster
- 81: Transmissionsverhalten von Augen-Hornhaut
- 82: Transmissionsverhalten von wässriger Augen-Hornhaut
- 83: Transmissionsverhalten von wässriger Augen-Hornhaut mit Linse
- 84: Transmissionsverhalten von wässriger Augen-Hornhaut mit Linse und Glaskörper
- 85: Gewebe
- 86: Schatten
- 87: geschwächter Bereich / einem Gelenk
- 88: Kraftrichtung
- 89: Vertiefung
- 90: Spalt / Einkerbung
- 91: belasteter Zustand
- 92: unbelasteter Zustand
- 93: Schraubenschlüssel
- 94: Schraubenkopf
- 95: erstes Bauelement
- 96: zweites Bauelement
- 97: erste Schiene
- 98: zweite Schiene
- 99: Messschieber
- 100: Markierung
- 101: Markierung

- x: x-Achse / laterale Richtung
- y: y-Achse / laterale Richtung
- z: z-Achse / axiale Richtung
- C: Kapazität
- t: Zeit
- I: Intensität
- D: Abstand
- T: Transmission
- λ: Wellenlänge
- d: Abstand von der Spitze

## Patentansprüche

1. Steuervorrichtung (1) für die Mikrochirurgie zur Steuerung eines chirurgischen Instruments (2),
**dadurch gekennzeichnet, dass**
die Steuervorrichtung (1) eine Sensoreinrichtung (3) umfasst, welche mindestens eine Einrichtung (4) zum Erfassen der lateralen Position des chirurgischen Instruments (2) relativ zu einer Oberfläche (12) eines Gewebes (11) und mindestens eine Einrichtung (5) zum Erfassen von Daten, welche eine axiale Beziehung zwischen dem chirurgischen Instrument (2) und der Gewebeoberfläche (12) kennzeichnen, wobei die axiale Beziehung durch mindestens einen kinematischen und/oder mindestens einen dynamischen Parameter charakterisiert ist, umfasst
und die Steuervorrichtung (1) zur Steuerung der Einwirkung des chirurgischen Instruments (2) auf das Gewebe (11) und/oder zur Steuerung der Bewegung des chirurgischen Instruments (2) in Bezug auf das Gewebe (11) basierend auf von der Sensoreinrichtung (3) erfassten Daten ausgelegt ist.

2. Steuervorrichtung (1) nach Anspruch 1,
**dadurch gekennzeichnet, dass**
es sich bei dem mindestens einen kinematischen Parameter um den Abstand zwischen dem chirurgischen Instrument (2) und der Gewebeoberfläche (12) in einer axialen Richtung (z) handelt, und/oder
es sich bei dem mindestens einen dynamischen Parameter um eine zwischen dem chirurgischen Instrument (2) und dem Gewebe (11) wirkende Kraft handelt.

3. Steuervorrichtung (1) nach Anspruch 1 oder Anspruch 2,
**dadurch gekennzeichnet, dass**
die mindestens eine Einrichtung (4) zum Erfassen der lateralen Position des chirurgischen Instruments (2) relativ zu der Gewebeoberfläche (12) mindestens eine Kamera zum gleichzeitigen Erfassen einer Vielzahl an lateral angeordneten Bildpunkten und/oder eine Scanvorrichtung zum zeitsequenziellen Erfassen lateral angeordneter Bildpunkte umfasst.

4. Steuervorrichtung (1) nach einem der Ansprüche 1 bis 3,
**dadurch gekennzeichnet, dass**
die mindestens eine Einrichtung (4) zum Erfassen der lateralen Position des chirurgischen Instruments (2) relativ zu der Gewebeoberfläche (12) und/oder die mindestens eine Einrichtung (5) zum Erfassen von Daten, welche eine axiale Beziehung zwischen dem chirurgischen Instrument (2) und der Gewebeoberfläche (12) kennzeichnen, eine Stereokamera und/oder einen optische Kohärenztomographen umfasst.

5. Steuervorrichtung (1) nach einem der Ansprüche 1 bis 4,
**dadurch gekennzeichnet, dass**
die mindestens eine Einrichtung (4) zum Erfassen einer axialen Beziehung zwischen dem chirurgischen Instrument (2) und der Gewebeoberfläche (12) mindestens einen in das chirurgische Instrument (2) integrierten Sensor und/oder
mindestens einen Sensor in Kombination mit mindestens einem zusätzlichen Aktuator an einer Schnittstelle zwischen einem Roboterarm (6) und dem chirurgischen Instrument (2) und/oder
mindestens eine Einrichtung zum Erfassen von Gewebereaktionen und/oder mindestens eine Einrichtung zur Umwandlung von durch mechanische Spannung erzeugter mechanischer Verformung des chirurgischen Instruments (2) in erfassbare optische Effekte umfasst.

6. Steuervorrichtung (1) nach einem der Ansprüche 1 bis 5,
**dadurch gekennzeichnet, dass**
die Steuervorrichtung (1) zur Herbeiführung und/oder Aufrechterhaltung einer Kraftausübung von dem chirurgischen Instrument (2) auf das Gewebe (11) ausgelegt ist,
und/oder die Steuervorrichtung (1) zur Herbeiführung einer Kraftausübung von dem chirurgischen Instrument (2) auf das Gewebe (11) ausgelegt ist, wobei die ausgeübte Kraft einen festlegbaren Schwellenwert nicht überschreitet.

7. Robotische Anordnung (9) für die Mikrochirurgie,
welche mindestens ein robotisch führbares chirurgisches Instrument (2) und mindestens einen robotischen Manipulator (6) mit einer Befestigungseinrichtung (7) zur Befestigung des chirurgischen Instruments (2) an dem robotischen Manipulator (6) umfasst,
**dadurch gekennzeichnet, dass**
die robotische Anordnung (9) eine Steuervorrichtung (1) nach einem der Ansprüche 1 bis 6 umfasst, welche zur Steuerung des chirurgischen Instruments (1) mittels des robotischen Manipulators (6) ausgelegt ist.

8. Robotische Anordnung (9) nach Anspruch 7,
**dadurch gekennzeichnet, dass**
der robotische Manipulator (6) mindestens 3 Bewegungsfreiheitsgrade zur Manipulation aufweist und die Steuervorrichtung (1) zur Steuerung der Bewegung des chirurgischen Instruments (2) mittels der mindestens 3 Bewegungsfreiheitsgrade des robotischen Manipulators (6) ausgelegt ist.

9. Robotische Anordnung (9) nach Anspruch 7 oder 8,
**dadurch gekennzeichnet, dass**
die robotische Anordnung (9) so ausgelegt, dass als ein Bezugspunkt für eine Bewegung des chirurgischen Instruments (2) ein Eintrittspunkt in ein bestimmtes Gewebe festgelegt oder festlegbar ist, wobei die laterale Position des chirurgischen Instruments (2) mit einer Winkelausrichtung des chirurgischen Instruments (2) oder des robotischen Manipulators (6) und die axiale Position des chirurgischen Instruments (2) mit einer Eintrittstiefe des chirurgischen Instruments (2) oder des robotischen Manipulators (6) durch den Eintrittspunkt korrespondiert.

10. Robotische Anordnung (9) nach einem der Ansprüche 7 bis 9,
**dadurch gekennzeichnet, dass**
die robotische Anordnung (9) zu einem teilautomatisierten oder vollautomatisierten Betrieb ausgelegt ist, wobei die lateralen Anteile einer Bewegungstrajektorie des chirurgischen Instruments (2) in Bezug auf die Gewebeoberfläche (12) basierend auf einer mittels der Sensoreinrichtung (3) erfassten lateralen Position steuerbar sind und/oder
der axiale Anteil der Bewegungstrajektorie des chirurgischen Instruments (2) in Bezug auf die Gewebeoberfläche (12) basierend auf einer mittels der Sensoreinrichtung (3) erfassten axialen Beziehung zwischen dem chirurgischen Instrument (2) und dem Gewebe (11) steuerbar ist.

11. Robotische Anordnung (9) nach einem der Ansprüche 7 bis 10,
**dadurch gekennzeichnet, dass**
die robotische Anordnung (9) eine Mensch-Maschine-Schnittstelle (13) umfasst, mittels welcher das chirurgische Instrument (2) bezüglich seiner lateralen Bewegung in Bezug auf das Gewebe (11) durch einen Nutzer steuerbar ist, während eine axiale Bewegung des chirurgischen Instruments (2) in Bezug auf das Gewebe (11) automatisiert steuerbar ist.

12. Robotische Anordnung (9) nach einem der Ansprüche 7 bis 11,
**dadurch gekennzeichnet, dass**
die robotische Anordnung (9) eine Mensch-Maschine-Schnittstelle (13) mit einer Planungsfunktion umfasst, mittels welcher basierend auf mittels der Sensoreinrichtung (3) erfassten lateralen Positionsdaten ein zu behandelnder Gewebebereich festlegbar ist, und die robotische Anordnung (9) dazu ausgelegt ist, einen festgelegten chirurgischen Schritt teilautomatisiert oder automatisiert basierend auf mittels der Sensoreinrichtung (3) erfassten Daten bezüglich der axialen Beziehung zwischen dem chirurgischen Instrument (2) und der Gewebeoberfläche (12) auszuführen,
und/oder die robotische Anordnung (9) eine Mensch-Maschine-Schnittstelle (13) zur Ausgabe von Informationen zur Assistenz umfasst.

13. Robotische Anordnung (9) nach einem der Ansprüche 7 bis 12,
**dadurch gekennzeichnet, dass**
die robotische Anordnung (9) während der Ausführung einer chirurgischen Maßnahme zwischen zwei Betriebsmodi umschaltbar ist, wobei in einem ersten Betriebsmodus das chirurgische Instrument (2) und das Gewebe (11) im Kontakt sind und in einem zweiten Betriebsmodus das chirurgische Instrument (2) und das Gewebe (11) nicht im Kontakt sind.

14. Robotische Anordnung (9) nach einem der Ansprüche 7 bis 13,
**dadurch gekennzeichnet, dass**
die robotische Anordnung (9) dazu ausgebildet ist, eine Bewegung des Gewebes (11) zu erfassen und im Falle einer erfassten Bewegung des Gewebes (11) mindestens das chirurgische Instrument (2) der erfassten Bewegung nachzuführen.

15. Operationsmikroskop (10) für die Mikrochirurgie,
welches eine Steuervorrichtung (1) nach einem der Ansprüche 1 bis 6 oder eine robotische Anordnung (9) nach einem der Ansprüche 7 bis 14 umfasst.
